# EUROPEAN PATENT SPECIFICATION

(11) **EP 3 362 927 B1**
(45) Date of publication and mention of the grant of the patent: **03.07.2024**
(21) Application number: 16782216.2
(22) Date of filing: 14.10.2016
(51) Int. Cl.: G16B 10/00, G16B 30/00, G16B 50/00, G16B 50/30

(54) **METHODS ASSOCIATED WITH A DATABASE THAT STORES A PLURALITY OF REFERENCE GENOMES**
VERFAHREN IM ZUSAMMENHANG MIT EINER DATENBANK, DIE EINE VIELZAHL VON REFERENZGENOMEN SPEICHERT
PROCÉDÉS ASSOCIÉS À UNE BASE DE DONNÉES CONSERVANT UNE PLURALITÉ DE GÉNOMES DE RÉFÉRENCE

(30) Priority: 16.10.2015 GB 201518364
(43) Date of publication of application: 22.08.2018
(73) Proprietor: Genome Research Limited, Hinxton, Saffron Walden CB10 1SA (GB)
(72) Inventor: LAWLEY, Trevor D., London Greater London NW1 2BF (GB); BROWNE, Hilary P., London Greater London NW1 2BF (GB); FORSTER, Sam C., London Greater London NW1 2BF (GB)
(74) Representative: Appleyard Lees IP LLP
(86) International application number: PCT/EP2016/074739
(87) International publication number: WO 2017/064263

(56) References cited:
- US-A1- 2014 045 744
- NICOLA SEGATA ET AL: "Metagenomic microbial community profiling using unique clade-specific marker genes", HHS PUBLIC ACCESS AUTHOR MANUSCRIPT, vol. 9, no. 8, 10 June 2012 (2012-06-10), pages 811-814, XP055309306, GB ISSN: 1548-7091, DOI: 10.1038/nmeth.2066
- T. A. K. FREITAS ET AL: "Accurate read-based metagenome characterization using a hierarchical suite of unique signatures", NUCLEIC ACIDS RESEARCH, vol. 43, no. 10, 12 March 2015 (2015-03-12), pages e69-e69, XP055331778, ISSN: 0305-1048, DOI: 10.1093/nar/gkv180
- SAMUEL C. FORSTER ET AL: "HPMCD: the database of human microbial communities from metagenomic datasets and microbial reference genomes", NUCLEIC ACIDS RESEARCH, vol. 44, no. D1, 17 November 2015 (2015-11-17), pages D604-D609, XP055331780, ISSN: 0305-1048, DOI: 10.1093/nar/gkv1216
- Mcnulty Nathan P. ET AL: "The Impact of a Consortium of Fermented Milk Strains on the Gut Microbiome of Gnotobiotic Mice and Monozygotic Twins", SCIENCE TRANSLATIONAL MEDICINE, vol. 3, no. 106, 26 October 2011 (2011-10-26), XP093121050, ISSN: 1946-6234, DOI: 10.1126/scitranslmed.3002701
- Mcnulty Nathan P. ET AL: "The Impact of a Consortium of Fermented Milk Strains on the Gut Microbiome of Gnotobiotic Mice and Monozygotic Twins - Supplementary Materials", SCIENCE TRANSLATIONAL MEDICINE, vol. 3, no. 106, 26 October 2011 (2011-10-26), XP093121054, ISSN: 1946-6234, DOI: 10.1126/scitranslmed.3002701
- Faith Jeremiah J. ET AL: "Predicting a Human Gut Microbiota's Response to Diet in Gnotobiotic Mice", Science, vol. 333, no. 6038, 1 July 2011 (2011-07-01), pages 101-104, XP093121047, US ISSN: 0036-8075, DOI: 10.1126/science.1206025
- J. J. FAITH ET AL: "Predicting a Human Gut Microbiota's Response to Diet in Gnotobiotic Mice - Supporting Online Material", SCIENCE, vol. 333, no. 6038, 19 May 2011 (2011-05-19), pages 101-104, XP055302156, US ISSN: 0036-8075, DOI: 10.1126/science.1206025

## Description

### FIELD OF THE INVENTION

The present invention relates to methods associated with a database that stores a plurality of reference genomes and phylogenetic information which relates the stored reference genomes to each other in a phylogenetic structure.

### BACKGROUND

Over the past decade, the application of metagenome sequencing to elucidate the microbial composition and functional capacity present in the human microbiome has revolutionized many concepts in our basic biology. The growing availability of metagenomic sequencing and associated analysis tools has enabled quantification and understanding of this microbial diversity at a level not previously possible.

The importance of understanding the tremendous molecular and phenotypic diversity present within a single bacterial lineage has also only recently become evident. Whole genome phylogeny of hundreds of isolates from a single "species" has demonstrated each "species" represents an evolutionary web of highly related lineages with diverse phenotypic characteristics. For example, strains of many opportunistic pathogen species including *Peptoclostridium difficile* and *Escherichia coli* that cannot be differentiated by 16S rRNA gene profiling, can range from benign members of the gastrointestinal tract to highly virulent pathogens, inducing severe, sometimes fatal symptoms in the host.

There are two primary types of metagenomic sequencing currently performed, 16S profiling and whole genome or "shotgun" metagenomic sequencing. These are described below:

### 16S Profiling

In this technique, a variable region of the highly conserved 16S gene is amplified and the resulting product subjected to high throughput sequencing. The resulting short reads from the 16S gene (typically ~100-150 base pairs) are then mapped (aligned) to a reference database using approaches such as the mothur pipeline (http://www.mothur.org/).

16S Profiling is highly efficient as all reads differentiate to the maximum level of phylogenetic resolution and this technique is standardized and widely used.

However, 16S profiling is limited to groups containing 16S gene (Bacteria and Archaea), and since the 16S gene is highly conserved, it is difficult to distinguish between different lineages at lower level branches of a phylogenetic tree. Resolution is therefore limited to distinguishing between groups (referred to as Operation Taxonomic Units, OTU) that differ in the small region of the 16S gene considered (typically Family or Genus level). That is, with 16S sequencing, deeper sequencing depth does not provide greater resolution.

Not all organisms have 16S gene, so these can't be identified by looking at the 16S gene and this, combined with bias potentially introduced by gene amplification, makes it difficult to determine the relative abundances of organisms present in a sample at a biologically meaningful level of resolution.

### Whole Genome Metagenomic Sequencing

Whole Genome metagenomic sequencing can be analyzed using *de-novo* assembly based approaches or using a lowest common ancestor approach. In this technique, the complete DNA sample (not just the 16S gene) is subjected to high throughput sequencing. The resulting short sequence reads (typically ~100-150 base pairs)

### Whole Genome Sequencing - De-novo assembly

In De-novo assembly based approaches reads are "assembled" by looking for regions that correspond to overlapping reads

Advantageously, De-novo assembly does not rely on reference genomes, thus overcoming issues associated with culturing.

However, De-novo assembly suffers from limited resolution when two genomes from closely related species are considered. Also, there is an inability to define complete genomic units, since De-novo assembly is limited to regions of the genome that are sequenced.

De-novo assembly is also extremely computationally intensive (so impractical for large datasets), and requires substantial sequence coverage to provide a useable dataset.

### Whole Genome Sequencing - Lowest Common Ancestor Approach

In this technique, the short reads (typically ~100-150bp) are assigned based on known reference genomes. This approach is best described in the Kraken algorithm publication (PMID: 24580807).

Advantageously, the lowest common ancestor approach allows fast classification of organisms present within a sample, and has improved resolution compared to 16S or De-*novo* assembly.

However, the lowest common ancestor approach is dependent on the quality and coverage of reference genomes.

The present inventors have observed that the lowest common ancestor approach generally provides information regarding the number of reads mapped to reference genomes in a sample from which short reads have been obtained, rather than the relative abundances of the reference genomes in the sample, thus limiting resolution and the ability to compare between species.

### Characterisation of the human microbiome

Most of our understanding of the human microbiome and its role in health and disease has primarily been derived from culture independent, genomic approaches, as described above.

It is now appreciated that, for example, the composition of the human intestinal microbiota is important for providing resistance to pathogen invasion (referred to as 'colonisation resistance') (Lawley and Walker 2013) and that, if the microbiota is perturbed (also referred to as 'dysbiosis'), the healthy base-line status can be restored through introduction of commensal intestinal bacteria (Petrof, Gloor et al. 2013, van Nood, Vrieze et al. 2013).

Attempts have been made to treat intestinal dysbiosis using faecal transplantation. Faecal transplantation involves transplanting intestinal bacteria from faeces of a healthy individual to an individual with an intestinal dysbiosis. This approach has been shown to provide an effective treatment for Clostridium difficile infection, for example (Petrof, Gloor et al. 2013, van Nood, Vrieze et al. 2013, Seekatz, Aas et al. 2014). However, faecal transplants have several drawbacks, including their undefined nature with respect to the bacteria and other microorganisms they contain, the availability of suitable donor material for large-scale clinical use, and administration of the faecal transplant to the patient. There thus remains a need in the art for defined bacterial mixtures for resolving dysbiosis and treatment of other diseases.

In order to utilise a bacterium that may be useful in resolving a dysbiosis or disease, the bacterium must first be isolated in culture, archived and characterised to ensure efficacy and safety. As the majority of the human microbiota is currently considered unculturable (Stewart 2012), this presents a significant limitation with regard to the bacteria which can be investigated and utilised as potential therapeutics.

One of the major limitations in culturing microbiota lies in characterising the bacteria present in a microbiota which have and/or have not been cultured using a particular set of culture conditions. Characterising bacteria successfully cultured using a set of culture conditions would allow the culture conditions to be used to prepare strain collections of the bacteria which could then be investigated for therapeutic applications. In addition, a means to identify bacteria not successfully cultured using a set of culture conditions would allow the culture conditions to be adjusted with a view to culturing bacteria of interest which were not successfully cultured initially. Methods for characterising the bacteria cultured from microbiota have been proposed (Goodman et al., 2011; US2014/045744). However, these methods rely on sequencing of the variable region 2 of the 16S ribosomal RNA (rRNA) gene and are thus not sufficiently sensitive to identify all of the species which were successfully isolated.

US2014/0045744A1 to the Washington University describes cultured collections of a gut microbial community, models comprising such cultures and methods of use thereof. In one example, a steady state mean absolute abundance of each community member was estimated for each of 36 mouse/diet combinations. In order to calculate the absolute abundance, relative abundances of microbes are calculated first. This is done by normalising read counts that uniquely identify a genome by the fraction of all possible k-mers the genome can produce that are unique.

The present invention has been devised in light of the above considerations.

### SUMMARY OF THE INVENTION

According to the invention, there is provided a method of analysing microorganisms present in a sample as defined in claim 1. Further features of the invention are provided in the dependent claims.

We use a database that stores a plurality of reference genomes and phylogenetic information which relates the stored reference genomes to each other in a phylogenetic structure.

For the purposes of this disclosure, a phylogenetic structure can be understood as a hierarchical structure which relates reference genomes to each other in one or more lineages, based on similarities/differences (e.g. genetic sequences that are present/not present) in the reference genomes. Computational techniques for inferring such a phylogenetic structure from stored reference genomes are well known, see e.g. Mauve (PMID: 15231754), Muscle (PMID: 15034147), and MAFFT (PMID: 23329690).

For the purpose of this disclosure, a lineage can be understood as a group of reference genomes inferred as being related to each other based on one or more similarities in the reference genomes (e.g. using a computational technique, as is known in the art).

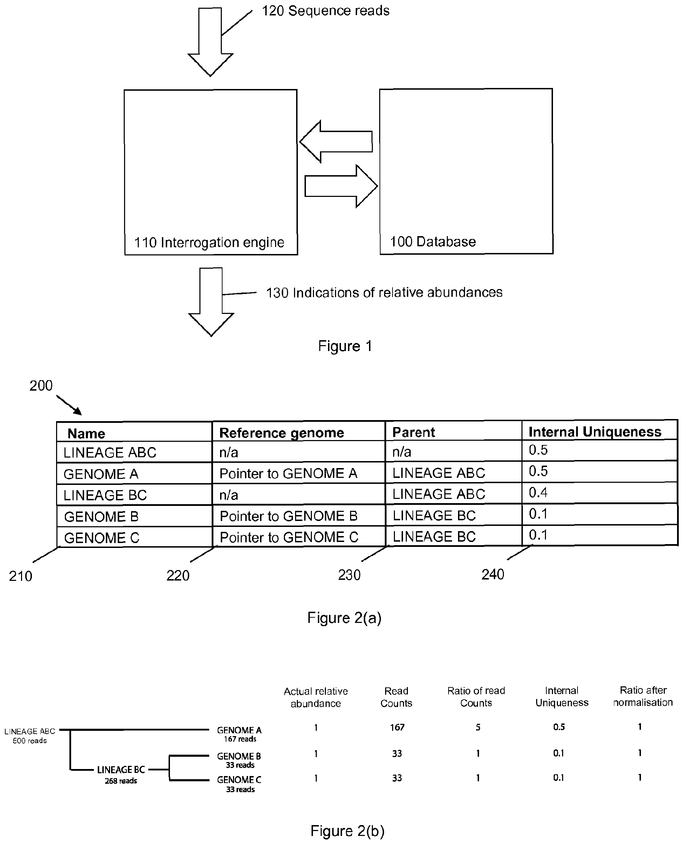

In the phylogenetic structure, each lineage/reference genome may be related to one or more other lineages/reference genomes according to a parent-child relationship. For the avoidance of any doubt, a lineage can be parent to one or more other lineages in the phylogenetic structure (see e.g. Fig. 2(a) and Fig. 2(b)).

A visualisation of a very simple example phylogenetic structure shown in Fig. 2(b), where "LINEAGE BC" is parent to "GENOME B" and "GENOME C", and "LINEAGE ABC" is parent to "GENOME A" and "LINEAGE BC". A lineage can thus be visualised as a branch of a phylogenetic tree

The method includes
using a plurality of sequence reads obtained from a sample to count the number of sequence reads deemed to uniquely map to each of a plurality of reference genomes within the phylogenetic structure;
for each of the plurality of reference genomes to which at least one sequence read has been deemed uniquely mapped, normalizing the number of sequence reads counted as being deemed uniquely mapped to the reference genome using a measure that reflects the uniqueness of the reference genome so as to obtain an indication of the relative abundance of the reference genome within the sample.

Thus, according to this method, indications of the relative abundances of reference genomes within the sample can be obtained. As discussed in more detail below, such values can be very useful in a range of 'downstream' applications.

In some cases, the method may include using a plurality of sequence reads obtained from a sample to count the number of sequence reads deemed as being uniquely mapped to only a subset of reference genomes within the phylogenetic structure, e.g. where that subset of reference genomes corresponds only to reference genomes of interest for a particular experimental study.

The method includes using a plurality of sequence reads obtained from a sample to count the number of sequence reads deemed as being uniquely mapped to a plurality of reference genomes (preferably all reference genomes) within the phylogenetic structure.

The method may include a preliminary step of inferring a phylogenetic structure from stored reference genomes, e.g. using a computational technique. As noted above, such computational techniques are well known, see e.g. Mauve (PMID: 15231754), Muscle (PMID: 15034147), and MAFFT (PMID: 23329690).

For the purposes of this disclosure, a measure of uniqueness of a reference genome is a measure that reflects (e.g. by being proportional to) the combined length of one or more genetic sequences deemed to uniquely identify the reference genome.

The method includes, for each of the plurality of reference genomes, determining a measure that reflects the uniqueness of the reference genome (e.g. so that the resulting measures can be used in normalizing the number of sequence reads counted as being deemed uniquely mapped to the reference genome, as described above).

The method includes, for each of the plurality of reference genomes, determining a measure that reflects the uniqueness of the reference genome by:
for the/each reference genome:
identifying one or more genetic sequences deemed to uniquely identify the reference genome;
determining a measure that reflects the uniqueness of the reference genome based on the one or more genetic sequences deemed to uniquely identify the reference genome.

As discussed in more detail below, identifying one or more genetic sequences deemed to uniquely identify a reference genome can potentially be computationally intensive. Therefore, the method may include storing each measure that reflects the uniqueness of a reference genome (or precursor of such a measure) in the database (e.g. in a uniqueness field of the database, as described below). In this way, the measure that reflects the uniqueness of a reference genome can be obtained more quickly when a sample is analysed, without having to re-perform the potentially computationally intensive task of identifying one or more genetic sequences deemed to uniquely identify a reference genome. More preferably, identifying one or more genetic sequences deemed to uniquely identify each of the plurality of reference genomes includes, for each reference genome in the database:
defining a plurality of segments, each segment containing a different genetic sequence from the reference genome;
for the genetic sequence contained in each segment:
   comparing the genetic sequence contained in the segment with the entirety of the genetic content of all other reference genomes in the database to establish whether the segment maps to any of the other reference genomes;
   if the genetic sequence contained in the segment maps to no other reference genome in the database, identifying the genetic sequence contained in the segment as being deemed to uniquely identify the reference genome.

Although potentially computationally intensive, comparing the genetic sequence contained in a segment with the entirety of the genetic content of all other reference genomes in the database to establish whether the segment maps to any of the other reference genomes is preferred, since this help to maximise resolution, i.e. helps to allow the identification of one or more genetic sequences that are deemed to uniquely identify closely related reference genomes

Preferably, identifying one or more genetic sequences deemed to uniquely identify each of the plurality of reference genomes is performed before the sequence reads are obtained from the sample, since these steps can be computationally intensive and do not require the sequence reads obtained from the sample in order to be performed.

Preferably, identifying one or more genetic sequences deemed to uniquely identify each of the plurality of reference genomes is performed each time a new reference genome is stored in the database, since adding a new reference genome to the database may cause a change in the genetic sequences identified as being deemed to uniquely identify a reference genome.

The plurality of segments defined for each reference genome may have a predetermined length, and preferably include each possible segment of that length that could be defined for the reference genome. The plurality of segments could be obtained using a sliding window technique, e.g. in which a window of predetermined length (e.g. 100 base pairs) is aligned with the start of the reference genome to define a first segment, and then the window is moved along the reference genome by a single base pair at a time to define further segments until each possible segment has been defined for the reference genome. Sliding window techniques are well known in the art.

The predetermined length of the segments may be chosen based on practical considerations, e.g. based on computational power/time required to perform calculations. Preferably, the predetermined length of the segments is chosen to be the same as the length of the sequence reads obtained from the sample (discussed below). However, the predetermined length of the segments could be selected from a wide range, e.g. 50-10,000+ base pairs.

Comparing the genetic sequence contained in a segment with all other reference genomes may be performed with an aligner, as is known in the art. In the examples below, a bowtie2 read aligner is used (see e.g. PMID: 22388286), though there are many other aligners that could be used such as bwa (see e.g. PMID 19451168).

For the avoidance of any doubt, a segment need not be identical to a reference genome in order for that sequence read to be established as being "mapped" to that reference genome, since it is known in the art that reference genomes may contain inaccuracies, e.g. introduced by the sequencing technologies used to derive the genetic sequences contained therein. Accordingly, any algorithm/aligner used to establish whether any segments map to a reference genome is configured to ignore minor differences of 2-3 base pairs for a segment 100 base pairs in length. As would be appreciated by a skilled person, ignoring minor differences in this way may result in minor differences caused by real mutations to be overlooked (i.e. so overlooked minor differences might not be limited to minor differences introduced by sequencing technologies).

Techniques for identifying one or more genetic sequences deemed to uniquely identify a reference genome other than those described above could be envisaged by a skilled person.

Using a plurality of sequence reads obtained from a sample to count the number of sequence reads deemed to uniquely map to each of a plurality of reference genomes within the phylogenetic structure includes:
for the/each reference genome:
comparing the plurality of sequence reads with the reference genome to establish whether any sequence reads map to the reference genome and to no other reference genome stored in the database;
if a sequence read maps to the reference genome and to no other reference genome stored in the database, counting the sequence read as being deemed to uniquely map to the reference genome.

Comparing the genetic sequence contained in a sequence read with all other reference genomes may be performed with an aligner, as is known in the art. In the example below, a bowtie2 read aligner is used (see e.g. PMID: 22388286), though there are many other aligners that could be used such as bwa (see e.g. PMID 19451168).

Comparing the plurality of sequence reads with each reference genome includes comparing each sequence read with the entirety of the genetic content of each reference genome. This allows more sequence reads to be uniquely mapped to reference genomes, compared with methods in which the minority of genetic content of the reference genomes are used. In contrast, many current comparison methods use small "marker sequences" that may represent less than 1% of genetic content within reference genomes.

Techniques for counting the number of sequence reads deemed to uniquely map to each of a plurality of reference genomes within a phylogenetic structure are known in the art (see e.g. PMID: 24580807).

A sequence read need not be identical to a reference genome in order for that sequence read to be established as being "mapped" to that reference genome, since it is known in the art that the sequence reads and reference genomes may contain inaccuracies, e.g. introduced by the sequencing technologies used to derive the genetic sequences contained therein. Accordingly, any algorithm/aligner used to establish whether any sequence reads map to a reference genome is configured to ignore minor differences, for a sequence read 100 base pairs in length, differences of 2-3 base pairs are ignored. Again, as would be appreciated by a skilled person, ignoring minor differences in this way may result in minor differences caused by real mutations to be overlooked (i.e. overlooked minor differences might not be limited to minor differences introduced by sequencing technologies).

Normalizing the number of sequence reads that were counted as being uniquely mapped to a reference genome of interest using a measure that reflects the uniqueness of that reference genome involves dividing the counted number of sequence reads by the measure.

Preferably, the database includes an entry for each reference genome within the phylogenetic structure.

Preferably, the entry for each reference genome includes a reference genome field for storing the reference genome or a pointer to the reference genome.

Preferably, the entry for each reference genome includes a parent field for storing a pointer to a parent of the reference genome within the phylogenetic structure.

Preferably, the entry for each reference genome includes a uniqueness field for storing a measure that reflects the uniqueness of the reference genome or a precursor of such a measure, which may have been determined as described above. As noted above, the measure or precursor stored in this field may allow the measure that reflects the uniqueness of the reference genome to be obtained more quickly when a sample is analysed, without having to re-perform the potentially computationally intensive task of identifying one or more genetic sequences deemed to uniquely identify a reference genome.

Since uniqueness can change when a new reference genome is stored in the database, the uniqueness field is preferably recalculated each time a new reference genome is stored in the database

The method may include obtaining the plurality of sequence reads from the sample, e.g. using a DNA sequencer.

Preferably, the sequence reads are obtained by a shotgun sequencing process, in which the DNA contained in the sample is broken up randomly into small segments which are then sequenced to obtain the plurality of sequence reads.

Preferably, the plurality of sequence reads from the sample are obtained from across the complete DNA of organisms within the sample (e.g. not just the 16S gene), e.g. whole genome shotgun sequencing.

The number of sequence reads obtained may be chosen using a measure that reflects the uniqueness of a reference genome of interest (e.g. determined as indicated above).

For example, the number of sequence reads to be obtained may be chosen to be at least m/u, where m is a minimum number of reads deemed appropriate for confident detection of a reference genome of interest, and where u is the internal uniqueness for that reference genome of interest, which represents the proportion of that individual reference genome of interest that is unique (relative to the genetic content of the individual reference genome). For a typical experiment, m is preferably 100 or more, more preferably 1000 or more.

The length of the sequence reads is preferably high enough to allow the sequence reads to be uniquely mapped to reference genomes in the database whilst being low enough to allow the sequence reads to be obtained with a high throughput.

Preferably, the sequence reads each have a length of at least 35 base pairs, more preferably 80 or more base pairs, so that random sequence reads can uniquely identify a reference genome. 100-150 base pairs would be typical with existing technologies. However, other lengths are plausible, and future sequencing technologies may result in other lengths becoming preferred.

The sample may be prepared to be suitable for DNA sequencing according to standard methods, known in the art.

In some embodiments, the reference genomes stored in the database may be (or may include) bacterial reference genomes.

We also describe an apparatus configured to perform a method as set out above.

The apparatus may include a computer configured (e.g. programmed) to perform a method as set out above (and/or any combination of steps set out above that involve manipulation of data).

We also describe a computer-readable medium having computer-executable instructions configured to cause a computer to perform a method as set out above (and/or any combination of steps set out above that involve manipulation of data).

We also describe methods which utilise a method as set out above.

By way of example, the method as defined in claim 1 may find utility in the analysis of bacteria present in a sample, the analysis of bacteria which have or have not been cultured using a microbial culturing method, methods of preparing culture collections of bacteria of interest, and methods of obtaining genomic sequences of bacteria of interest.

In addition, the method as defined in claim 1 may find utility in identifying therapeutic bacteria, and in the diagnosis of diseases characterised by the presence of a bacterium. These and other methods are described below.

A sample, as referred to herein, may be a sample obtained from any source which is expected to comprise a microorganism, such as a bacterium. The sample may thus be a sample comprising a microorganism, e.g. a bacterium. Samples comprising microorganisms, including bacteria, can be obtained from many sources, including humans, animals, and environmental sources, such as soil samples.

In a preferred embodiment, the sample is obtained from an individual, i.e. a human individual. In this case, the sample may be a microbiota sample. Microbiota in this context refers to the microorganisms that are present on and in an individual. For example, intestinal microbiota and skin microbiota refer to the microbiota present in the intestine and on the skin of an individual, respectively.

The individual from whom a sample has been obtained may be, for example, a healthy individual or an individual with a disease or dysbiosis, as applicable. Dysbiosis may refer to an imbalance in the microbiota of an individual, and has been implicated in a number of diseases and disorders, such as inflammatory bowel disease (IBD).

The sample may be a body fluid or solid matter, or tissue biopsy, such as a faecal sample, a urine sample, a skin scrape, a colon biopsy, a lung biopsy, or a skin biopsy. In one example, the sample may be a faecal sample.

Where the context requires, the sample may be an uncultured sample, i.e. a sample which has not been subjected to any culturing, such as bacterial culturing. This is important in the context of identifying microorganisms, such as bacteria, present in the microbiota of an individual, for example.

For the purpose of this disclosure, a bacterial lineage, which is present e.g. in a sample, can be understood as a group of bacteria with genomes inferred as being related to each other based on one or more similarities in the genomes (e.g. using a computational technique, as is known in the art).

We also describe a method of analysing the bacteria present in a sample, wherein the method includes:
obtaining a plurality of sequence reads from (e.g. by performing whole genome shotgun sequencing of DNA extracted from) (i) a first portion of a sample obtained from an individual and using a method according to the invention to obtain indications of the relative abundances of one or more reference genomes within the first portion of the sample;
obtaining a plurality of sequence reads from (e.g. by performing whole genome shotgun sequencing of DNA extracted from) (ii) bacteria cultured from a second portion of the sample using a bacterial culturing method and using a method according to the invention to obtain indications of the relative abundances of one or more reference genomes within the cultured portion of the sample; and
comparing the indications of the relative abundances of the reference genomes within the first portion of the sample with the indications of the relative abundance of the reference genomes within the cultured sample.

Methods for obtaining a plurality of sequence reads, such as whole genome shotgun sequencing, are known in the art and are described elsewhere herein. Methods for extracting DNA from a sample are similarly known.

The described method of analysing the bacteria present in a sample may be a method of determining the bacteria present in a sample obtained from an individual which have or have not been cultured using a bacterial culturing method, wherein the method includes:
determining the bacteria present in the first portion of the sample which were and/or were not cultured using the bacterial culturing method by comparing the indications of the relative abundances of the reference genomes within the first portion of sample with the indications of the relative abundance of the reference genomes within the cultured sample.

This may find application, for example, in determining the proportion (e.g. percentage) of bacteria present in a sample, such as a microbiota sample obtained from an individual which have or have not been cultured using a bacterial culturing method. This may be useful, for example, when formulating or developing a broad range medium for culturing bacteria from a sample, such as a microbiota sample, or comparing different broad range media with respect to the proportion (e.g. percentage) of bacteria from a sample, such as a microbiota sample, whose growth the medium can support.

The described method of analysing the bacteria present in a sample may thus be a method of determining the proportion (e.g. percentage) of bacteria present in a sample, such as a sample obtained from an individual, which have or have not been cultured using a bacterial culturing method, wherein the method includes:
determining the proportion (e.g. percentage) of bacteria present in the first portion of the sample which were and/or were not cultured using the bacterial culturing method by comparing the indications of the relative abundances of the reference genomes within the first portion of sample with the indications of the relative abundance of the reference genomes within the cultured sample.

The described method of analysing the bacteria present in a sample may be used in the context of determining the bacteria which have been cultured using multiple, alternate, bacterial culturing methods. An alternate bacterial culturing method in this context refers to a different bacterial culturing method. The bacterial culturing methods may either be performed in parallel, using multiple portions of the same sample, or sequentially using different samples. In the latter case, the samples are preferably obtained from the same source, for example the same individual, to allow comparison between the bacteria successfully cultured using each culturing method. Alternate bacterial culturing methods may be used as part of a process for culturing the majority of the bacteria present in a sample, as different bacteria present in a sample are likely to have different growth requirements.

Where the bacterial culturing methods are employed sequentially, this may be as part of an iterative process for culturing the majority of the bacteria present in a sample, in which the bacteria present in a first sample which were and/or were not cultured using a first bacterial culturing method are determined, followed by determining the bacteria present in a second sample, preferably obtained from the same source at the first sample, e.g. the same individual, which were and/or were not cultured using a second, alternate, bacterial culturing method, and optionally determining the bacteria present in a second portion of the sample which were cultured using the second bacterial culturing method but which were not cultured using the first bacterial culturing method. This process can be repeated until the majority of the bacteria present in the sample have been cultured. This is useful, for example, for preparing culture collections of bacteria reflecting the majority of the bacteria present in a microbiome obtained from an individual, e.g. a healthy individual, or obtaining genomic sequences of the majority of the bacteria present in a microbiome obtained from a healthy individual and/or an individual with a dysbiosis.

A "majority" in this context may refer to culturing of at least 70%, at least 75%, at least 80%, at least 85%, at least 90%, at least 91%, at least 92%, at least 93%, at least 94%, at least 95%, at least 96%, at least 97%, at least 98%, or at least 99% of the bacterial present in a sample. "Bacteria" in this context may refer to a bacterial genus or a bacterial species. The present inventors have shown, for example, that using the broad range medium YCFA, with and without prior ethanol treatment and addition of taurocholate, bacteria from 97% of the most abundant genera and 90% of the most abundant species detected in a sample using metagenomics shotgun sequencing could be isolated. It is expected that reiteration of the culturing step using an alternate medium would allow an even higher proportion (e.g. percentage) of the bacterial genera and bacterial species present in a sample to be cultured.

The described method of analysing the bacteria present in a sample may therefore further comprise:
obtaining a plurality of sequence reads from (e.g. by performing whole genome shotgun sequencing of DNA extracted from) (iii) bacteria cultured from a second sample, preferably obtained from the same source, e.g. the same individual, as the sample in (i) and (ii), using an alternate bacterial culturing method and using a method according to the invention to obtain indications of the relative abundances of one or more reference genomes within the alternately cultured sample; and
comparing the indications of the relative abundances of the reference genomes within the alternately cultured sample with the indications of the relative abundance of the reference genomes within the first portion of the sample and, optionally, the indications of the relative abundance of the reference genomes within the cultured sample.

Alternatively, where alternate bacterial culturing methods are used sequentially, the described method of analysing the bacteria present in a sample may comprise:
obtaining a plurality of sequence reads from (e.g. by performing whole genome shotgun sequencing of DNA extracted from) (iii) bacteria cultured from a third portion of the sample using an alternate bacterial culturing method and using a method according to the invention to obtain indications of the relative abundances of one or more reference genomes within the alternately cultured sample; and
comparing the indications of the relative abundances of the reference genomes within the alternately cultured sample with the indications of the relative abundance of the reference genomes within the first portion of the sample and, optionally, the indications of the relative abundance of the reference genomes within the cultured sample.

In this case, the described method of analysing the bacteria present in a sample may be a method of determining the bacteria present in a sample obtained from an individual which have been cultured using an alternate bacterial culturing method, wherein the method includes:
(a) determining the bacteria present in the sample which were and/or were not cultured using the alternate bacterial culturing method by comparing the indications of the relative abundances of the reference genomes within the alternately cultured sample with the indications of the relative abundance of the reference genomes within the first portion of the sample; or
(b) determining the bacteria present in the sample which were cultured using the alternate bacterial culturing method and which were not cultured with the bacterial culturing method by comparing the indications of the relative abundances of the reference genomes within the alternately cultured sample with the indications of the relative abundance of the reference genomes within the first portion of the sample and the relative abundance of the reference genomes within cultured sample.

An alternate bacterial culturing method may be specifically adapted for, or specifically selected for, culturing bacteria which were not cultured with a first bacterial culturing method. Bacterial culturing methods for many bacterial families, genera and species are known in the art, as are methods for adapting a bacterial culturing method to culture bacteria from a particular bacterial family, genus, or species of interest. Similarly, many bacterial culturing methods, and methods for adapting bacterial culturing methods, to culture bacteria with a particular genotype and/or phenotype of interest are known. By identifying one or more bacteria of interest which were not cultured using a bacterial culturing method, thus allows a bacterial culturing method to be selected for, or the bacterial culturing method adapted for, culturing said bacteria of interest. Again this is useful, for example, for preparing culture collections of bacteria reflecting the majority of the bacteria present in a microbiome obtained from an individual, e.g. a healthy individual, or obtaining genomic sequences of the majority of the bacteria present in a microbiome obtained from a healthy individual and/or an individual with a dysbiosis.

Culture collections of bacteria of interest are useful for a number of different applications. For example, culture collections representing bacteria from the human microbiome may serve as a repository of potential candidates for bacteriotherapy of a disease or dysbiosis

The described method of analysing the bacteria present in a sample may thus be a method of preparing a culture collection of bacteria of interest present in a sample obtained from an individual, the method including identifying a bacterial culturing method for culturing the bacteria of interest, wherein the method includes:
determining the bacteria of interest present in the sample which were cultured using the bacterial culturing method by comparing the indications of the relative abundances of the reference genomes within the first portion of sample with the indications of the relative abundance of the reference genomes within the cultured sample; and
employing the bacterial culturing method to prepare a collection of cultures of said bacteria of interest from the sample.

The cultures are preferably pure cultures of bacteria. A pure culture may be a culture of a single bacterium.

Many bioinformatics approaches require the genomic sequence of a bacterium of interest to be known. For example, genomic sequences of bacteria can be compiled into databases which can then interrogated. Methods for whole genome sequencing are known in the art.

The described method of analysing the bacteria present in a sample may therefore be a method of obtaining the genomic sequence of one or more bacteria of interest present in a sample obtained from an individual, wherein the method includes:
determining the bacteria of interest present in the sample which were cultured using the bacterial culturing method by comparing the indications of the relative abundances of the reference genomes within the first portion of sample with the indications of the relative abundance of the reference genomes within the cultured sample;
employing the bacterial culturing method to prepare cultures of one or more of the bacteria of interest from the sample, and
determining the genomic sequence(s) of said bacteria.

The genomic sequence(s) of one or more bacteria of interest obtained using a method of the invention may be stored (e.g. as a new reference genome) in a database that stores reference genomes (e.g. a reference database as described above). Thus, the coverage of a database that stores reference genomes (e.g. a reference database as described above) can be improved by the methods described herein. As noted below, the methods according to the invention may be found particularly effective when used with a database that provides thorough genome coverage in an area of interest (e.g. an area which reflects the expected content of a sample of interest).

The worldwide emergence of bacterial resistance to antibacterial agents has produced a need for new methods of combating bacterial infections. The use of (harmless) bacteria to displace or inhibit pathogenic bacteria, such as *Clostridium difficile,* has been investigated for this purpose.

In addition, it is now thought that dysbiosis plays a role in a number of diseases, including inflammatory bowel disease. The administration of (harmless bacteria, e.g. through faecal transplanatation, represents a promising approach for treating (resolving) dysbiosis. However, treatment regimens such a faecal transplantation have a number of disadvantages, including their undefined nature with respect to the bacteria and other microorganisms they contain, the availability of suitable donor material for large-scale clinical use, and administration of the faecal transplant to the patient.

There thus remains a need in the art for identifying bacteria which can be used in the treatment of diseases characterised by the presence of a pathogenic bacterium and for the treatment of dysbiosis.

We also describe a method of identifying a bacterium for bacteriotherapy for a dysbiosis, the method comprising:
obtaining a plurality of sequence reads from (e.g. by performing whole genome shotgun sequencing of DNA extracted from) a sample obtained from a patient with the dysbiosis;
using a method according to the invention to obtain indications of the relative abundances of reference genomes within the sample;
comparing the indications of the relative abundances of the reference genomes within the sample with the relative abundance of the reference genomes in a control;
selecting a bacterium with a genome which is absent from the sample obtained from the patient but present in the control, or which is present at a lower relative abundance in the sample obtained from the patient compared with the control, for bacteriotherapy for the dysbiosis.

A patient as referred to herein is preferably a human patient.

A lower relative abundance of a bacterium may refer to a relative abundance which less than 50%, less than 40%, less than 30%, less than 20%, less than 10%, less than 5%, less than 4%, less than 3%, less than 2%, or less than 1% of the relative abundance of the bacterium in the control.

Alternatively, a lower relative abundance of a bacterium may refer to a relative abundance which is 2-fold or more, 5-fold or more, 10-fold or more, 20-fold or more, 30-fold or more, 40-fold or more, 50-fold or more, or 100-fold or more lower than the relative abundance of the bacterium in the control.

The control in this context may be a sample obtained from an individual without the dysbiosis, e.g. a healthy individual, or a group of such individuals. Alternatively, the control may be a reference value for the expected abundance of the bacterium in an individual without the dysbiosis.

A dysbiosis may refer to an imbalance in the microbiota of an individual. An imbalance in this context may refer to a disruption in the normal diversity and/or function of the microbiota. For example, dysbiosis may refer to an imbalance in, such as disruption in the normal diversity and/or function of, the commensal bacteria of an individual. A dysbiosis may be associated with one or more (disease) symptoms or may be symptomless.

Dysbiosis is thought to play a role in a number of diseases and syndromes, including: inflammatory bowel disease (IBD) (such as Crohn's Disease and ulcerative colitis); cancer (including colorectal cancer); enteric microbial infections, such as enteric bacterial infections (including *Clostridium difficile* infections), enteric viral infections, or enteric fungal infections; hepatic encephalopathy; asthma; Parkinson's disease, multiple sclerosis, autism, irritable bowel syndrome (IBS), coeliac disease, allergies, metabolic syndrome, cardiovascular disease, and obesity.

We also describe a method of identifying a bacterium for bacteriotherapy for a dysbiosis, the method comprising:
obtaining a plurality of sequence reads from (e.g. by performing whole genome shotgun sequencing of DNA extracted from) (i) a first sample obtained from a patient with the dysbiosis;
obtaining a plurality of sequence reads from (e.g. by performing whole genome shotgun sequencing of DNA extracted from) (ii) a second sample obtained from the same patient after the patient has received a faecal transplant;
using a method according to the invention to obtain indications of the relative abundances of reference genomes within the sample;
comparing the indications of the relative abundances of the reference genomes within the first sample with the relative abundance of the reference genomes in the second sample;
selecting a bacterium with a genome which is absent from the first sample but present in the second sample, or which is present at a lower relative abundance in the first sample compared with the second sample, for bacteriotherapy for the dysbiosis.

Methods for faecal transplantation are known in the art. The faecal transplant is a faecal transplant from an individual without the dysbiosis, e.g. a healthy individual.

We also describe a method of identifying a bacterium for therapy of a disease characterised by the presence of a pathogenic bacterium, the method comprising:
obtaining a plurality of sequence reads from (e.g. by performing whole genome shotgun sequencing of DNA extracted from) (i) a first sample obtained from a first asymptomatic carrier of the pathogenic bacterium, and obtaining a plurality of sequence reads from (e.g. by performing whole genome shotgun sequencing of DNA extracted from) (ii) a second sample obtained from a second asymptomatic carrier of the pathogenic bacterium;
using a method according to the invention, to obtain indications of the relative abundances of reference genomes within the first sample and the second sample;
comparing the indications of the relative abundances of the reference genomes within the first sample with the relative abundance of the reference genomes in the second sample;
selecting a bacterium with a genome which is common to the first and second sample for bacteriotherapy for the disease.

A bacterium which is common to the first and second samples, as referred to above, may be present in the first and second samples at the same, or substantially the same, abundance.

An asymptomatic carrier in this context may refer to an individual who is infected with a pathogenic bacterium but exhibits no disease symptoms normally associated with the pathogenic bacterium.

We also describe a method of identifying a bacterium for therapy of a disease characterised by the presence of a pathogenic bacterium, the method comprising:
obtaining a plurality of sequence reads from (e.g. by performing whole genome shotgun sequencing of DNA extracted from) (i) a first sample obtained from an asymptomatic carrier of the pathogenic bacterium, and obtaining a plurality of sequence reads from (e.g. by performing whole genome shotgun sequencing of DNA extracted from) (ii) a second sample obtained from healthy individual;
using a method according to the invention to obtain indications of the relative abundances of reference genomes within the first sample and the second sample;
comparing the indications of the relative abundances of the reference genomes within the first sample with the relative abundance of the reference genomes in the second sample;
selecting a bacterium with a genome which is present in the first sample but absent from the second sample, or which is present at a higher relative abundance in the first sample compared with the second sample, for bacteriotherapy for the disease.

A higher relative abundance of a bacterium may refer to a relative abundance which is 50% or more, 100% or more, 500% or more, or 1000% or more higher than the relative abundance of the bacterium in the second sample.

Alternatively, a higher relative abundance of a bacterium may refer to a relative abundance which is 2-fold or more, 5-fold or more, 10-fold or more, 20-fold or more, 30-fold or more, 40-fold or more, 50-fold or more, 100-fold or, 500-fold or more, or 100-fold or more higher than the relative abundance of the bacterium in the second sample.

Many disease characterised by the presence of pathogenic bacteria are known in the art and include *Clostridium difficile* infection, and methicillin-resistant Staphylococcus aureus (MRSA) infection.

We also describe a bacterium identified using a method of identifying a bacterium for bacteriotherapy for a dysbiosis or disease as described above, wherein the bacterium is for use in a method of treating the dysbiosis or disease in an individual.

We also describe a bacterium for use in a method of treating a dysbiosis or disease in a patient, the method comprising identifying the bacterium using a method of identifying a bacterium for bacteriotherapy for a dysbiosis or disease as described above, and administering the identified bacterium to a patient.

We also describe a method of treating a dysbiosis or disease in an individual, the method comprising identifying the bacterium using a method of identifying a bacterium for bacteriotherapy for a dysbiosis or disease as described above, and administering a therapeutically effective amount of the identified bacterium to the patient.

Many diseases present with similar symptoms in the clinic and identifying the causative agent of such disease can be time-consuming and difficult, increasing costs and leading to delays for the patient until a suitable treatment can be administered. One example of such a disease is diarrhoeal disease which can be caused by many different microorganisms. It would therefore be advantageous if the causative agent of such diseases could be more easily identified.

We also describe a method of diagnosing a disease in a patient, the method comprising:
obtaining a plurality of sequence reads from (e.g. by performing whole genome shotgun sequencing of DNA extracted from) a sample obtained from a patient;
using a method according to the invention to obtain indications of the relative abundances of reference genomes within the sample;
comparing the indications of the relative abundances of the reference genomes within the sample with the relative abundance of the reference genomes in a control;
identifying a bacterium with a genome which is present in the sample obtained from the patient but absent from the control, or which is present at a higher relative abundance in the sample obtained from the patient compared with the control;
wherein the presence, or higher relative abundance, of the bacterium in the sample is indicative of the disease.

A higher relative abundance of a bacterium may refer to a relative abundance which is 50% or more, 100% or more, 500% or more, or 1000% or more higher than the relative abundance of the bacterium in the control.

Alternatively, a higher relative abundance of a bacterium may refer to a relative abundance which is 2-fold or more, 5-fold or more, 10-fold or more, 20-fold or more, 30-fold or more, 40-fold or more, 50-fold or more, 100-fold or, 500-fold or more, or 100-fold or more higher than the relative abundance of the bacterium in the control.

The control in this context may be a sample obtained from a healthy individual, or a group of healthy individuals. Alternatively, the control may be a reference value for the expected abundance of the bacterium in a healthy individual.

A method of diagnosing a disease in a patient may further comprise:
selecting the individual for treatment for the disease; or
subjecting an individual for treatment for the disease.

The treatment may be any known treatment for the disease in question.

We also describe a diagnostic system for use in a method according to the invention, the system comprising:
a tool or tools for obtaining a plurality of sequence reads from (e.g. by performing whole genome shotgun sequencing of DNA extracted from) a sample obtained from a patient; and
a computer configured (e.g. programmed) to obtain indications of the relative abundances of reference genomes within the sample using a method according to the invention from the plurality of sequence reads obtained from the sample (which may be genome shotgun sequencing data).

We also describe a method of treating a disease in a patient, the method comprising:
(i) requesting a test providing the results of an analysis, the test including:
   obtaining a plurality of sequence reads from (e.g. by performing whole genome shotgun sequencing of DNA extracted from) a sample obtained from a patient;
   using a method according to the invention to obtain indications of the relative abundances of reference genomes within the sample;
   comparing the indications of the relative abundances of the reference genomes within the sample with the relative abundance of the reference genomes in a control;
   identifying a bacterium with a genome which is present in the sample obtained from the patient but absent from the control, or which is present at a higher relative abundance in the sample obtained from the patient compared with the control;
   wherein the presence, or higher relative abundance, of the bacterium in the sample is indicative of the disease;
(ii) treating the individual for the disease.

We also describe a method of identifying the bacterial causative agent of a disease in a patient, the method comprising:
obtaining a plurality of sequence reads from (e.g. by performing whole genome shotgun sequencing of DNA extracted from) a sample obtained from a patient;
using a method according to the invention, to obtain indications of the relative abundances of reference genomes within the sample;
comparing the indications of the relative abundances of the reference genomes within the sample with the relative abundance of the reference genomes in a control;
identifying a bacterium with a genome which is present in the sample obtained from the patient but absent from the control, or which is present at a higher relative abundance in the sample obtained from the patient compared with the control;
wherein said bacterium is the causative agent of the disease.

We also describe a method of analysing the bacteria and/or bacteria lineages present in a sample wherein the method includes performing whole genome shotgun sequencing.

The described method of analysing the bacteria and/or bacteria lineages present in a sample may provide a method analysing the bacteria and/or bacterial lineages present in a sample, wherein the method includes:
performing whole genome shotgun sequencing of (i) DNA extracted from a first portion of the sample and (ii) DNA extracted from bacteria cultured from a second portion of the sample using a bacterial culturing method;
identifying one or more reference genomes and/or lineages in a database to which at least one of the plurality of sequence reads obtained in (i) is deemed to uniquely map, wherein the database stores a plurality of reference genomes and phylogenetic information which relates the stored reference genomes to each other in a phylogenetic structure;
identifying one or more reference genomes and/or lineages in the database to which at least one of the plurality of sequence reads obtained in (ii) is deemed to uniquely map;
comparing the one or more reference genomes and/or lineages to which at least one of the plurality of sequence reads obtained in (i) were deemed to uniquely map with the one or more reference genomes and/or lineages to which at least one of the plurality of sequence reads obtained in (ii) were deemed to uniquely map.

Optionally, this method may comprise identifying all reference genomes and/or lineages in a database to which at least one of the plurality of sequence reads obtained in (i) is deemed to uniquely map; and
identifying all reference genomes and/or lineages in the database to which at least one of the plurality of sequence reads obtained in (ii) is deemed to uniquely map.

The described method of analysing the bacteria and/or bacteria lineages present in a sample may be a method of determining the bacteria and/or bacterial lineages present in a sample obtained from an individual which have or have not been cultured using a bacterial culturing method, wherein the method includes:
determining the bacteria and/or bacterial lineages present in the first portion of the sample which were and/or were not cultured using the bacterial culturing method by comparing the one or more reference genomes and/or lineages to which at least one of the plurality of sequence reads obtained in (i) were deemed to uniquely map with the one or more reference genomes and/or lineages to which at least one of the plurality of sequence reads obtained in (ii) were deemed to uniquely map.

This method may find application in, for example, determining the proportion (e.g. percentage) of bacteria and/or bacterial lineages present in a sample, such as a sample obtained from an individual which have or have not been cultured using a bacterial culturing method. This may be useful, for example, when formulating or developing a broad range medium for culturing bacteria from a sample, or comparing different broad range media with respect to proportion (e.g. percentage) of bacteria from a sample whose growth the medium can support.

The described method of analysing the bacteria and/or bacteria lineages present in a sample may thus be a method of determining the proportion (e.g. percentage) of bacteria present in a sample obtained from an individual which have or have not been cultured using a bacterial culturing method, wherein the method includes:
determining the proportion (e.g. percentage) of bacteria and/or bacterial lineages present in the first portion of the sample which were and/or were not cultured using the bacterial culturing method by comparing the reference genomes and/or lineages to which at least one of the plurality of sequence reads obtained in (i) were deemed to uniquely map with the reference genomes and/or lineages to which at least one of the plurality of sequence reads obtained in (ii) were deemed to uniquely map.

The described method of analysing the bacteria and/or bacteria lineages present in a sample may be used in the context of determining the bacteria which have been cultured using multiple, alternate, bacterial culturing methods. An alternate bacterial culturing method in this context refers to a different bacterial culturing method. The bacterial culturing methods may either be performed in parallel, using multiple portions of the same sample, or sequentially using different samples. In the latter case, the samples are preferably obtained from the same source, for example the same individual, to allow comparison between the bacteria successfully cultured using each culturing method. Alternate bacterial culturing methods may be used as part of a process for culturing the majority of the bacteria present in a sample, as different bacteria present in a sample are likely to have different growth requirements.

Where the bacterial culturing methods are employed sequentially, this may be as part of an iterative process for culturing the majority of the bacteria present in a sample, in which the bacteria and/or bacterial lineages present in a first sample which were and/or were not cultured using a first bacterial culturing method are determined, followed by determining the bacteria and/or bacterial lineages present in a second sample, preferably obtained from the same source at the first sample, e.g. the same individual, which were and/or were not cultured using a second, alternate, bacterial culturing method, and optionally determining the bacteria and/or bacterial lineages present in a second portion of the sample which were cultured using the second bacterial culturing method but which were not cultured using the first bacterial culturing method. This process can be repeated until the majority of the bacteria present in the sample have been cultured. This is useful, for example, for preparing culture collections of bacteria reflecting the majority of the bacteria present in a microbiome obtained from a healthy individual, or obtaining genomic sequences of the majority of the bacteria present in a microbiome obtained from a healthy individual and/or an individual with a dysbiosis.

A "majority" in this context may refer to culturing of at least 70%, at least 75%, at least 80%, at least 85%, at least 90%, at least 91%, at least 92%, at least 93%, at least 94%, at least 95%, at least 96%, at least 97%, at least 98%, or at least 99% of the bacterial present in a sample. "Bacteria" in this context may refer to a bacterial genus or a bacterial species. The present inventors have shown, for example, that using the broad range medium YCFA, with and without prior ethanol treatment and addition of taurocholate, bacteria from 97% of the most abundant genera and 90% of the most abundant species detected in a sample using metagenomics shotgun sequencing could be isolated. It is expected that reiteration of the culturing step using an alternate medium would allow an even higher proportion (e.g. percentage) of the bacterial genera and bacterial species present in a sample to be cultured.

The described method of analysing the bacteria and/or bacteria lineages present in a sample may therefore further comprise:
performing whole genome shotgun sequencing of (iii) DNA extracted from bacteria cultured from a second sample, preferably obtained from the same source, e.g. the same individual, as the sample in (i) and (ii), using an alternate bacterial culturing method;
identifying one or more reference genomes and/or lineages in the database to which at least one of the plurality of sequence reads obtained in (iii) is deemed to uniquely map;
comparing the one or more reference genomes and/or lineages to which at least one of the plurality of sequence reads obtained in (iii) were deemed to uniquely map with the one or more reference genomes and/or lineages to which at least one of the plurality of sequence reads obtained in (i) were deemed to uniquely map and, optionally, the one or more reference genomes and/or lineages to which at least one of the plurality of sequence reads obtained in (ii) were deemed to uniquely map.

Alternatively, where alternate bacterial culturing methods are used in parallel for example, the described method of analysing the bacteria and/or bacteria lineages present in a sample may further comprise:
performing whole genome shotgun sequencing of (iii) DNA extracted from bacteria cultured from a third portion of the sample using an alternate bacterial culturing method;
identifying one or more reference genomes and/or lineages in the database to which at least one of the plurality of sequence reads obtained in (iii) is deemed to uniquely map;
comparing the one or more reference genomes and/or lineages to which at least one of the plurality of sequence reads obtained in (iii) were deemed to uniquely map with the one or more reference genomes and/or lineages to which at least one of the plurality of sequence reads obtained in (i) were deemed to uniquely map and, optionally, the one or more reference genomes and/or lineages to which at least one of the plurality of sequence reads obtained in (ii) were deemed to uniquely map.

In this case, the described method of analysing the bacteria and/or bacteria lineages present in a sample may be a method of determining the bacteria and/or bacterial lineages present in a sample obtained from an individual which have been cultured using an alternate bacterial culturing method, wherein the method includes:
(a) determining the bacteria and/or bacterial lineages present in the sample which were and/or were not cultured using the alternate bacterial culturing method by comparing the one or more reference genomes and/or lineages to which at least one of the plurality of sequence reads obtained in (iii) were deemed to uniquely map with the one or more reference genomes and/or lineages to which at least one of the plurality of sequence reads obtained in (i) were deemed to uniquely map; or
(b) determining the bacteria and/or bacterial lineages present in the sample which were cultured using the alternate bacterial culturing method and which were not cultured with the bacterial culturing method by comparing the one or more reference genomes and/or lineages to which at least one of the plurality of sequence reads obtained in (iii) were deemed to uniquely map with the one or more reference genomes and/or lineages to which at least one of the plurality of sequence reads obtained in (ii) were deemed to uniquely map and the one or more reference genomes and/or lineages to which at least one of the plurality of sequence reads obtained in (i) were deemed to uniquely map.

The described method of analysing the bacteria and/or bacteria lineages present in a sample may be a method of preparing a culture collection of bacteria of interest present in a sample obtained from an individual, the method including identifying a bacterial culturing method for culturing the bacteria of interest, wherein the method includes:
determining the bacteria of interest present in the sample which were cultured using the bacterial culturing method by comparing the one or more reference genomes and/or lineages to which at least one of the plurality of sequence reads obtained in (i) were deemed to uniquely map with the one or more reference genomes and/or lineages to which at least one of the plurality of sequence reads obtained in (ii) were deemed to uniquely map; and
employing the bacterial culturing method to prepare a collection of cultures of said bacteria of interest from the sample.

The cultures are preferably pure cultures of bacteria. A pure culture may be a culture of a single bacterium.

In addition, the described method of analysing the bacteria and/or bacteria lineages present in a sample may be a method of obtaining the genomic sequence of one or more bacteria of interest present in a sample obtained from an individual, wherein the method includes:
determining the bacteria of interest present in the sample which were cultured using the bacterial culturing method by comparing the one or more reference genomes and/or lineages to which at least one of the plurality of sequence reads obtained in (i) were deemed to uniquely map with the one or more reference genomes and/or lineages to which at least one of the plurality of sequence reads obtained in (ii) were deemed to uniquely map;
employing the bacterial culturing method to prepare cultures of one or more of the bacteria of interest from the sample, and
determining the genomic sequence(s) of said bacteria.

The genomic sequence(s) of one or more bacteria of interest obtained using a method of the invention may be stored (e.g. a new reference genome) in a database that stores reference genomes (e.g. a reference database as described above). Thus, the coverage of a database that stores reference genomes (e.g. a reference database as described above) can be improved by the methods described herein. As noted below, the methods according to the invention may be found particularly effective when used with a database that provides thorough genome coverage in an area of interest (e.g. an area which reflects the expected content of a sample of interest).

The above described methods have been described with respect to bacteria and bacterial lineages. However, it is expected that these aspects are equally applicable to microorganisms other than bacteria, including fungi and viruses, such as bacteriophages. In this context, "microorganism" may thus refer to a bacterium, fungus, or virus. For example, it is known that microorganisms other than bacteria a present in samples obtained from humans, animals, and environmental sources, such as soil samples, as described above. DNA can be extracted from such microorganisms, or samples comprising microorganisms, and a plurality of sequence reads obtained therefrom, e.g. by performing whole genome shotgun sequencing, and analysed as described herein.

Any reference in the description of the methods above to a bacterium or bacteria may thus be replaced with a reference to a microorganism or microoganisms, a fugus or fungi, or a virus or viruses (such as a bacteriophage or bacteriophages), as applicable.

Similarly, any reference to a bacterial lineage in the description of the methods above may be replaced with a reference to a microbial lineage, a fungal lineage or a viral lineage. For the purpose of this disclosure, a microbial lineage, which is present e.g. in a sample, can be understood as a group of microorganisms (such as a group of bacteria, fungi, or viruses) with genomes inferred as being related to each other based on one or more similarities in the genomes (e.g. using a computational technique, as is known in the art). A fungal lineage, which is present e.g. in a sample, can be understood as a group of fungi with genomes inferred as being related to each other based on one or more similarities in the genomes (e.g. using a computational technique, as is known in the art), and a viral lineage, which is present e.g. in a sample, can be understood as a group of viruses with genomes inferred as being related to each other based on one or more similarities in the genomes (e.g. using a computational technique, as is known in the art).

References to bacterial culturing methods in the description of the methods above may accordingly also be replaced with references to microbial culturing methods, fungal culturing methods, or viral culturing methods, as applicable. Methods for culturing many microorganisms are known, including methods for culturing fungi and viruses.

Where the description of the described method of analysing the bacteria present in a sample refers to a method of identifying a bacterium for "bacteriotherapy" for a dysbiosis, this may be replaced with a reference to "therapy", where the method is a method of identifying a microorganism, fungus, or virus for treatment of a dysbiosis or disease. In particular, use of bacteriophages for therapy is contemplated. Similarly, where this method refers to identifying the "bacterial causative agent" of a disease, this may be replaced with "microbial causative agent", "fungal causative agent", or "viral causative agent".

By way of example, the described method of analysing the bacteria present in a sample may thus provide:
a method of analysing the microorganisms present in a sample, wherein the method includes:
obtaining a plurality of sequence reads from (e.g. by performing whole genome shotgun sequencing of DNA extracted from) (i) a first portion of a sample obtained from an individual and using a method according to the first aspect of the invention to obtain indications of the relative abundances of one or more reference genomes within the first portion of the sample;
obtaining a plurality of sequence reads from (e.g. by performing whole genome shotgun sequencing of DNA extracted from) (ii) microorganisms cultured from a second portion of the sample using a microbial culturing method and using a method according to the invention, to obtain indications of the relative abundances of one or more reference genomes within the cultured portion of the sample; and
comparing the indications of the relative abundances of the reference genomes within the first portion of the sample with the indications of the relative abundance of the reference genomes within the cultured sample.

Similarly, the described method of analysing the bacteria and/or bacteria lineages present in a sample may thus provide:
a method analysing the microorganisms and/or microbial lineages present in a sample, wherein the method includes:
performing whole genome shotgun sequencing of (i) DNA extracted from a first portion of the sample and (ii) DNA extracted from microorganisms cultured from a second portion of the sample using a microbial culturing method;
identifying one or more reference genomes and/or lineages in a database to which at least one of the plurality of sequence reads obtained in (i) is deemed to uniquely map, wherein the database stores a plurality of reference genomes and phylogenetic information which relates the stored reference genomes to each other in a phylogenetic structure;
identifying one or more reference genomes and/or lineages in the database to which at least one of the plurality of sequence reads obtained in (ii) is deemed to uniquely map;
comparing the one or more reference genomes and/or lineages to which at least one of the plurality of sequence reads obtained in (i) were deemed to uniquely map with the one or more reference genomes and/or lineages to which at least one of the plurality of sequence reads obtained in (ii) were deemed to uniquely map.

### BRIEF DESCRIPTION OF THE DRAWINGS

Examples of these proposals are discussed below, with reference to the accompanying drawings in which:
Fig. 1 shows an example method of using a database that stores a plurality of reference genomes and phylogenetic information which relates the stored reference genomes to each other in a phylogenetic structure.
Fig. 2(a) shows the content of a simplified example of a database that stores three reference genomes and phylogenetic information which relates the stored reference genomes to each other in a phylogenetic structure.
Fig. 2(b) shows the phylogenetic structure of the database shown in Fig. 2(a).
Fig. 3 shows the relative proportions of species reported from a sample containing equal proportions of bacterial species for kraken approach, read counts normalized to genome size (which represents the present inventors' method prior to the present invention), read counts normalized by genome uniqueness, actual percentages.
Fig. 4 shows the relative proportions of species reported from a sample containing mixed proportions of bacterial species for kraken approach, read counts normalized to genome size (which represents the present inventors' method prior to the present invention), read counts normalized by genome uniqueness, actual percentages.
Fig. 5 shows a comparison of bacteriotherapy candidates predicted to provide protection against *Clostridium difficile* identified through widescale analysis of co-occurrence from the Database (red) and the RePOOPULATE Study (PMCID: PMC3869191) (blue).
Fig. 6 and Fig. 7 are drawings relating to the HPMC database described in Annex A.
Fig. 8, Fig. 9 and Fig. 10 are drawings setting out an example workflow described in Annex B.
Fig. 11 shows a schematic work-flow for a process comprising identifying bacteria and/or bacterial lineages present in a faecal sample which have/have not been cultured using a set of bacterial culture conditions, adjusting the culture conditions as necessary to culture bacteria not cultured using an original set of bacterial culture conditions, obtaining the whole genome sequence and/or cultures of bacteria cultured using a suitable set of bacterial culture conditions. The steps can be performed together or separately, as applicable.

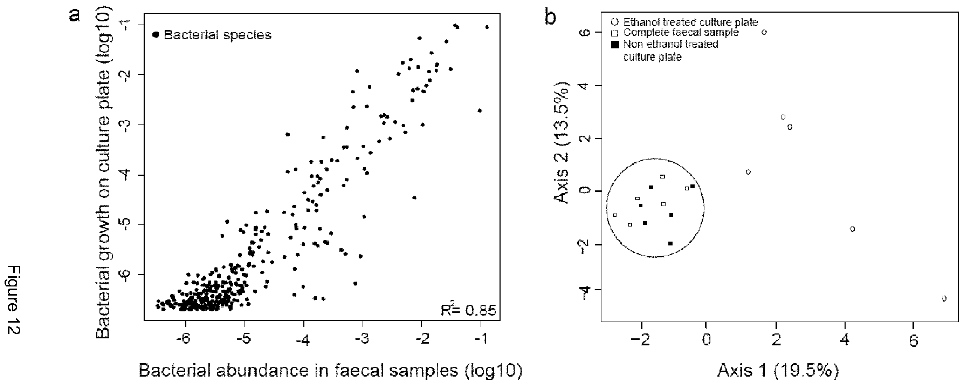

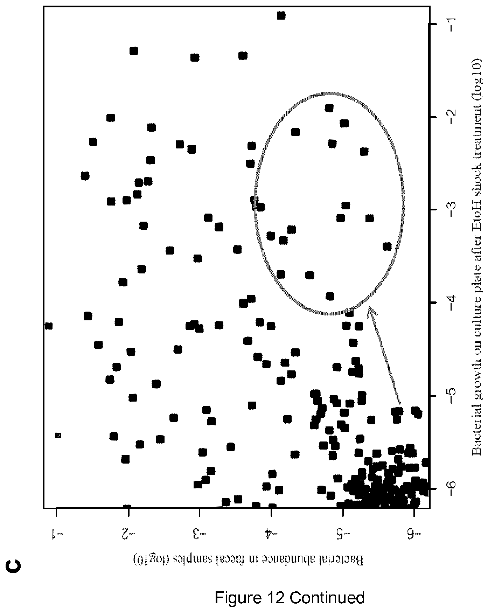

Fig. 12: Targeted phenotypic culturing facilitates bacterial discovery from healthy human faecal microbiota. Fig. 12 (a) shows the relative abundance of bacteria in faecal samples (x axis) compared to relative abundance of bacterial species growing on YCFA agar plates (y axis) as determined by metagenomic sequencing. Bacteria grown on YCFA agar are representative of the complete faecal samples as indicated by R²=0.85. Fig. 12 (b) shows a principal components analysis (PCoA) plot of 16S rRNA gene sequences detected from 6 donor faecal samples representing bacteria in the complete faecal samples (unfilled squares), faecal bacterial colonies recovered from YCFA agar plates without ethanol pre-treatment (filled black squares) or with ethanol pre-treatment to select for ethanol resistant spore forming bacteria (circles). Culturing without ethanol selection is representative of the complete faecal sample, ethanol treatment shifts the profile, enriching for ethanol resistant spore forming bacteria and allowing their subsequent isolation. Fig. 12 (c) shows the relative abundance of bacteria grown on a culture plate after ethanol shock treatment (x axis) compared to the relative abundance of bacteria in the original faecal sample (y axis). Ethanol shock treatment of the faecal sample before culturing increased the proportion of spore forming bacteria that subsequently grew on the culture plate (as circled), allowing their isolation. Each dot represents a bacterial species.

### DETAILED DESCRIPTION

In general, the following discussion describes examples of our proposals that provide a method suitable for quantifying relative species and strain abundance from high-throughput metagenomic sequencing samples. This is achieved through specific normalization methods in the context of high quality reference genomes.

The example method shown in Fig. 1 is implemented by a database 100 and an interrogation engine 110 configured to interrogate the database 100.

The database 100 stores a plurality of reference genomes and phylogenetic information which relates the stored reference genomes to each other in a phylogenetic structure.

The interrogation engine 110 uses a plurality of sequence reads 120 obtained from a sample to count the number of sequence reads deemed to uniquely map to each of a plurality of reference genomes within the phylogenetic structure.

As described in more detail below, the interrogation engine 110, for each of the plurality of reference genomes to which at least one sequence read has been deemed uniquely mapped, normalizes the number of sequence reads counted as being deemed uniquely mapped to the reference genome using a measure that reflects the uniqueness of the reference genome so as to obtain a value indicative of the relative abundance of the reference genome within the sample, thereby obtaining indications of relative abundances 130.

In the practical example discussed below, the database 100 is the HPMC database described in more detail in Annex A.

However, to provide a reader with a better understanding of the methods described herein, illustrate a method of using the database 100 in accordance with the invention, a simplified example of a database 200 is illustrated in Fig. 2(a) and the phylogenetic structure of the database 200 is illustrated in Fig. 2(b). An theoretical example showing how the database 200 can be used to provide indications of relative abundances using sequence reads obtained from a sample is provided below.

As shown in Fig. 2(a), the database 200 includes an entry for each reference genome and each lineage within the phylogenetic structure.

As shown in Fig. 2(a), the entry for each lineage/reference genome includes a name field 210 for storing a name by which the entry can be referenced.

As shown in Fig. 2(a), the entry for each reference genome further includes a reference genome field 220 for storing the reference genome or a pointer to the reference genome (this entry is blank for entries corresponding to lineages). A pointer to the reference genome is preferred since the reference genomes tend to be large in size.

As shown in Fig. 2(a), the entry for each reference genome further includes a parent field 230 for storing a pointer to a parent of the lineage/reference genome within the phylogenetic structure.

The content of the parent fields can be viewed as phylogenetic information which relates the stored reference genomes to each other in a phylogenetic structure, since an entire phylogenetic tree can be constructed from the information contained in the parent fields. Of course, phylogenetic information could be stored in numerous other ways, as would be appreciated by a skilled person.

Computational techniques for inferring such a phylogenetic structure from stored reference genomes are well known. For the HPMC database described below, the present inventors used the 16S/18S sequence to define the broad tree with closely related species resolved through whole genome alignment (preferably an on-going exercises within the database) e.g. using Mauve (PMID: 15231754), Muscle (PMID: 15034147), or MAFFT (PMID: 23329690).

As shown in Fig. 2(a), the entry for each lineage/reference genome further includes a uniqueness field 240 for storing an internal uniqueness value, which represents the proportion of the individual lineage or reference genome that is unique (relative to the genetic content of the individual lineage or reference genome).

The internal uniqueness value for each entry is calculated by identifying one or more genetic sequences deemed to uniquely identify the corresponding reference genome (if the entry is a reference genome), and then dividing the combined length of these sequences by the length of the corresponding reference genome (if the entry is a reference genome). Techniques for identifying one or more genetic sequences deemed to uniquely identify a reference genome have already been described in detail above.

Identifying one or more genetic sequences deemed to uniquely identify each reference genome in the database includes, for each reference genome in the database:
defining a plurality of segments, each segment containing a different genetic sequence from the reference genome;
for the genetic sequence contained in each segment:
   comparing the genetic sequence contained in the segment with the entirety of the genetic content of all other reference genomes in the database to establish whether the segment maps to any of the other reference genomes;
   if the genetic sequence contained in the segment maps to no other reference genome in the database, identifying the genetic sequence contained in the segment as being deemed to uniquely identify the reference genome.

In the present examples, the plurality of segments were obtained using a sliding window technique of length 100 base pairs and comparing the genetic sequence contained in a segment with all other reference genomes was performed with bowtie2 read aligner (see e.g. PMID: 22388286).

Referring back now to Fig. 1, the plurality of sequence reads 120 obtained from a sample are used to count the number of sequence reads deemed to uniquely map to each reference genome 100 within the phylogenetic structure stored as phylogenetic information in the database 100. Techniques for counting the number of sequence reads deemed to uniquely map to each reference genome have already been discussed above. In the present examples, the counting utilises a bowtie2 read aligner (see e.g. PMID: 22388286).

Next, for each of the plurality of reference genomes to which at least one sequence read has been deemed uniquely mapped, the interrogation engine 110 normalizes (by dividing) the number of sequence reads counted as being deemed uniquely mapped to the reference genome using a measure that reflects the uniqueness of the reference genome so as to obtain a value indicative of the relative abundance of the reference genome within the sample, thereby obtaining indications of relative abundances 130.

As discussed in more detail below, where all of the reference genomes stored in the database are similar or equal in length (as is assumed to be the case for the database 200 of Fig. 2(a)), the internal uniqueness value can be used as a measure that reflects the uniqueness of the corresponding reference genome.

However, as discussed in more detail below, where all of the reference genomes stored in the database are unequal in length, the internal uniqueness value is preferably adjusted (e.g. "on the fly" by the interrogation engine 110) based on the length of the corresponding reference genome (if the entry is a reference genome) in order to provide a measure that reflects the uniqueness of the corresponding reference genome. In this case, the internal uniqueness value stored in the database can be viewed as a precursor to a measure that reflects the uniqueness of the corresponding reference genome.

Storing an internal uniqueness value in the uniqueness field 240 of the database can be useful in analyses which are not the focus of this disclosure, since this value allows a direct comparison of the percentage uniqueness between reference genomes and lineages of different lengths. Nonetheless, in other embodiments (not exemplified herein), the uniqueness field 240 of the entry for each reference genome could instead store an "global" uniqueness value that is proportional to the combined length of one or more genetic sequences deemed to uniquely identify the corresponding reference genome. In this case, the "global" uniqueness value could be used as the measure that reflects the uniqueness of the corresponding reference genome regardless of whether all of the reference genomes stored in the database are equal/unequal in length, thereby avoiding any need to adjust the internal uniqueness value "on the fly" where reference genomes stored in the database are unequal in length.

Methods described herein may be viewed as extending on the lowest common ancestor approach described in the "Background" section, above. Given thorough genome coverage provided by the HPMC database described in Annex A (which prior to the HPMC database most people would not have), a problem with applying existing approaches to uniquely classify the content of a given sample to reference genomes using the HPMC database is that very few reads could uniquely be mapped to closely related reference genomes. Adding more reference genomes to the HPMC database is helpful to identify/reduce/avoid inaccurate classification, but further reduces the number of reads that can be uniquely classified to reference genomes, especially if reference genomes share a large proportion of their genetic content (consider the extreme case of a single nucleotide polymorphism, "SNP", between two reference genomes: only sequence reads from a sample that cover that SNP could be used to distinguish the two reference genomes in the sample).

To correct for this problem, methods described herein use a measure that reflects the uniqueness of each reference genome, thereby taking into account the uniqueness of each reference genome, so as to obtain indications of the relative abundances of reference genomes within a sample.

Indications of relative abundances determined according to a method as described herein may be utilised in a number of different downstream applications. An example workflow in which the indications of relative abundances determined according to a method as described herein may be used is shown in Annex B.

### Theoretical example using the database of Fig. 2(a)

The following theoretical example, which is provided to provide a reader with a better understanding of the methods described herein, uses the simplified database 200 of Fig. 2(a).

Fig. 2(b) provides a representation of the phylogenetic structure of the reference genomes stored in database 200.

In the example of Fig. 2(a) and Fig. 2(b), there are three reference genomes, which have internal uniqueness as follows:
GENOME A: 0.5
GENOME B: 0.1
GENOME C: 0.1

From this, and the phylogenetic information shown in Fig. 2(b), it can be inferred that GENOME A shares 50% of its genome with GENOME B and GENOME C, and GENOME B and C share 90% of their respective genomes with each other.

For simplicity, GENOME A, GENOME B and GENOME C are assumed to have the same length.

Starting with Sample A that has equal representation of each genome. Random sequence reads from Sample A should result in an equal number of sample reads being uniquely mapped to each genome.

However, due to the differing uniquenesses of the three genomes, sequence reads from Sample A will not be uniquely mapped to the three genomes in equal numbers.

For example, when you classify 1000 sequence reads from Sample A, one would expect:
~ 500 to map to each of GENOME A, GENOME B or GENOME C and therefore be uniquely mapped to LINEAGE ABC (since these sequence reads can't distinguish between the 50% of genome shared between GENOME A, B and C)
~ 166 to map uniquely to GENOME A
~ 268 to map to each of GENOME B and GENOME C and therefore be uniquely mapped to LINEAGE BC (since these sequence reads can't distinguish between the 90% of genome shared between GENOME B and C)
~ 33 to map uniquely to GENOME B
~ 33 to map uniquely to GENOME C.

By counting the number of sequence reads deemed to uniquely map to each genome, the total sequence reads for each genome would be reported as:
GENOME A: ~166 (71.5% uniqueness at genome level, 33.2% of total assigned) - 5
GENOME B: ~33 (14.2% uniqueness at genome level, 6.6% of total assigned) - 1
GENOME C: ~33 (14.2% uniqueness at genome level, 6.6% of total assigned) - 1

However, normalizing the counted number using internal uniqueness values provides a more accurate indication of the real relative abundances present in Sample A and allows direct comparison between all genomes and lineages in the database 200:
GENOME A: (166)/(0.5), relative abundance - 1
GENOME B: (33)/(0.1), relative abundance - 1
GENOME C: (33)/(0.1), relative abundance - 1

Fig. 2(b) has been annotated with the numbers discussed above.

In the above calculations, internal uniqueness (which represents the proportion of the individual reference genome that is unique, relative to the genetic content of the individual reference genome) is used as a measure that reflects the uniqueness of the reference genome.

However, if the genomes were not equal in length, then the internal uniqueness value is preferably adjusted (e.g. "on the fly") based on the length of the corresponding reference genome to provide a measure that reflects the uniqueness of the lineage or reference genome, which would adjust the above calculation as follows:
GENOME A: (no. reads)/(0.5 × I_{A}), relative abundance - 1
GENOME B: (no. reads)/(0.1 × I_{B}), relative abundance - 1
GENOME C: (no. reads)/(0.1 × I_{C}), relative abundance - 1

Where I_{A} is the length of GENOME A, I_{B} is the length of GENOME B, Ic is the length of GENOME C.

Obviously, if the database 200 were to incorporate many more reference genomes and many more sequence reads, the internal uniqueness of the reference genomes might drop. However, a fundamental benefit of the normalization approach is it allow one to adjust the read counts so that indications of relative abundances can be obtained.

Importantly, the method is not limited to obtaining relative abundances of reference genomes in a sample.

For example if for Sample A one wished to compare the relative abundance of GENOME A to LINEAGE BC, once could perform the following calculations:
GENOME A: (166)/(0.5), relative abundance - 1
LINEAGE BC: (268)/(0.4), relative abundance - 2

Thus, the above-exemplified method provides the ability to compare relative abundances of any genome or lineage combination through normalizing the counted numbers of sequence reads uniquely mapped to those genomes and/or lineages.

The method also works regardless of the starting composition of the sample.

For example, considering Sample B that has unequal representation of each genome in a ratio of 1:2:2 (GENOME A : GENOME B : GENOME C) the total sequence reads for each genome would be reported as:
GENOME A: ~100 (55.6% uniqueness at genome level, 20.0% of total assigned) - 5
GENOME B: ~40 (22.2% uniqueness at genome level, 8.0% of total assigned) - 2
GENOME C: ~40 (22.2% uniqueness at genome level, 8.0% of total assigned) - 2

Again, normalizing the counted number using internal uniqueness values provides a more accurate indication of the real relative abundances present in Sample B:
GENOME A: (100)/(0.5), relative abundance ~ 1
GENOME B: (40)/(0.1), relative abundance - 2
GENOME C: (40)/(0.1), relative abundance - 2

Similarly comparing GENOME A to LINEAGE BC:
GENOME A: (100)/(0.5), relative abundance ~ 1
LINEAGE BC: (320)/(0.4), relative abundance - 2

The accuracy of the genome/lineage identification and quantification is fundamentally dependent on the quality of available reference genomes in the database. As described with reference to a practical example below, the HPMC database described in Annex A, which was populated with reference genomes using techniques described in this application, can be used to provide useful results in the case of gut flora. Without access to a database storing a comprehensive collection of reference genomes relevant to a sample under study, results may be less useful.

Assuming the database provides a comprehensive collection of reference genomes, the resolution of classification may be limited by sequencing depth. Accordingly, the number of sequence of sequence reads to be obtained may be chosen to be at least m/u, where m is a minimum number of reads deemed appropriate for confident detection of a lineage or reference genome of interest, and where u is the internal uniqueness, which represents the proportion of the individual lineage or reference genome that is unique (relative to the genetic content of the individual lineage or reference genome). For a typical experiment, m is preferably 100 or more, more preferably 1000 or more.

### Practical Example using the HPMC database described in Annex A - Controlled data

To demonstrate the practical effectiveness of the methods described herein, it is possible to considered 5 species *Aspergillus fumigatus, Bifidobacterium breve 689b, Bifidobacterium breve S27, Clostridium difficile 630 and Staphylococcus phage K.* This selection simultaneously demonstrates the method is effective on eukaryotic components of the microbiota (*Aspergillus*) with large genomes (29.3Mb) and bacteriophage *(Staphylococcus phage K,* 0.01Mb genome). It also demonstrates the ability to differentiate the two strains of 8. *breve* (genome size ~2.3Mb) and a distantly related bacteria C. *difficile.*

To demonstrate the effectiveness of this method it is necessary to utilize real sequencing reads to capture variability observed in real sequence reads. However, in this case it is not possible to know the "true" metagenomic content of a metagenomic sample. To overcome this limitation sequencing reads obtained from direct genome sequencing are sampled at a prescribed percentage to generate pseudo-metagenomic sequencing reads at known proportions.

The measure used to normalize counts is essential to the method, but the specific form of the measure and the detail with which it is calculated is not important for the method's success, so long as it reflects the uniqueness of the lineages and/or reference genomes being studied.

For this example, the uniqueness measure used to normalize counts was calculated by using a 100bp sliding window approach. The genome and lineage uniqueness used to normalize counts was reported as the percentage of 100bp regions that would uniquely identify the genome or lineage against all other genomes within the database. The comparison was performed using the bowtie2 algorithm with standard parameters. Read abundance levels were then weighted by this measure as described above to determine the relative species abundance from the relative read abundance.

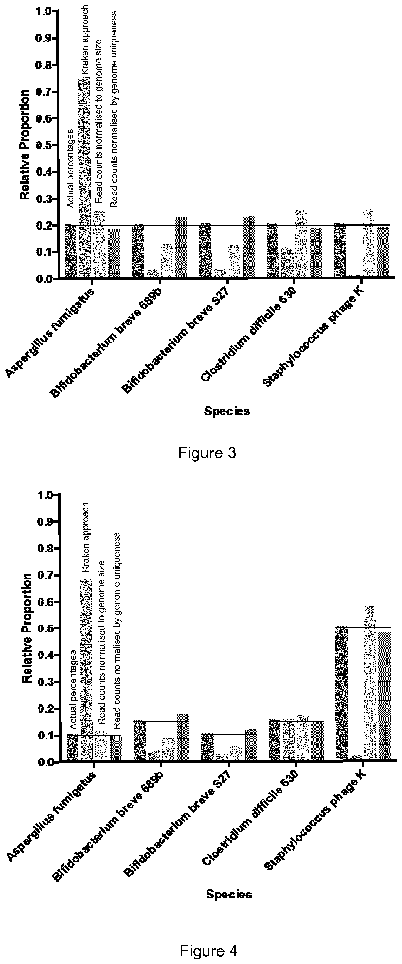

Sample containing equal proportions (Fig. 3):

| | |
|---|---|
| 1:1:1:1:1 | Aspergillus : *B*. *breve* 689b : *B*. *breve* S27 : *C. difficile* 630 : *Saphylococcus phage K* |

Sample containing mixed proportions (Fig. 4):

| | |
|---|---|
| 2:3:2:3:10 | Aspergillus : *B*. *breve* 689b : *B*. *breve* S27 : *C*. *difficile* 630 *Saphylococcus phage K* |

Sequence reads were randomly selected from the complete genome sequences of each species and assembled into a pseudo-metagenomic sample with known read proportions. Read abundance levels are then weighted by this "uniqueness factor" as described above to determine the relative species abundance from the relative read abundance.

Applying the uniqueness normalization to the sample containing equal proportions:

| | |
|---|---|
| Aspergillus: | ∼ 1 |
| *B*. *breve* 689b: | ∼ 1 |
| *B*. *breve* S27: | ∼ 1 |
| *C*. *difficile* 630: | ∼ 1 |
| *Saphylococcus phage K:* | ∼ 1 |

Applying the uniqueness normalization to the sample containing mixed proportions:

| | |
|---|---|
| Aspergillus: | ∼ 2 |
| *B. breve* 689b: | ∼ 3 |
| *B. breve* S27: | ∼ 2 |
| *C. difficile* 630: | ∼ 3 |
| *Saphylococcus phage K:* | ∼ 10 |

It is also possible to calculate the relative abundances of any particular lineage using this method. In this example there are two strains of B. *breve* represented. Considering these two strains as a single 8. *breve* lineage, uniqueness normalization for the sample containing equal proportions provides results as follows:

| | |
|---|---|
| Aspergillus: | ∼ 1 |
| *B*. *breve* Lineage: | ∼ 2 |
| *C*. *difficile* 630: | ∼ 1 |
| *Saphylococcus phage K:* | ∼ 1 |

Using uniqueness normalization for the sample containing equal proportions provides results as follows:

| | |
|---|---|
| Aspergillus: | ∼ 2 |
| *B*. *breve* Lineage: | ∼ 5 |
| *C*. *difficile* 630: | ∼ 3 |
| *Saphylococcus phage K:* | ∼ 10 |

Note that calculating relative abundance for a lineage involves counting the number of sequence reads deemed to uniquely map to the lineage and normalizing that count using a measure that reflects the uniqueness of the lineage, rather than just adding the relative abundances determined for individual members of the lineage (though the result should come out as similar - as above - assuming that there is good coverage of the lineage in the database).

### Practical Example using the HPMC database described in Annex A - Real data

To demonstrate the practical benefits of this approach it is possible to consider many examples where identification of specific species or strains can provide important insights to biology or bacteriotherapy candidate design as it provide exact species or strains as opposed to genera or family level approximations.

### Example 1: Bacteriotherapy Candidate Identification

One specific example is the identification of *C. difficile* bacteriotherapy candidates. When applying this analysis approach to 435 public metagenomic samples where *C. difficile* is detected in individuals that report normal health it is possible to also identify commonly co-occurring species that are likely to play a role in maintaining health and preventing uncontrolled *C. difficile* expansion (and thus disease). This analysis identifies 30 species that commonly associate with asymptomatic *C. difficile carriers* (p < 0.01). When compared to the publicly available RePOOPULATE study (PMC3869191) 24 of the 25 species identified were represented in this list (*Eubacterium desmolans* was absent).

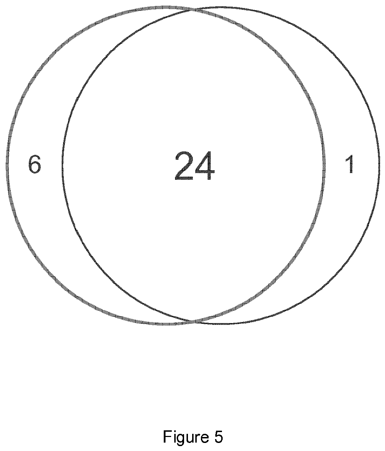

Fig. 5 shows a comparison of bacteriotherapy candidates predicted to provide protection against *Clostridium difficile* identified through widescale analysis of co-occurrence from the Database (left) and the RePOOPULATE Study (PMCID: PMC3869191) (right).

### Example 2: Biomarker Identification

Accurate, species and strain level pathogen and commensal identification will provide an important tool for metagenomic based diagnostics and biomarker identification. The proposed method could be utilized to identify the specific strains of a particular pathogen, such as identification of epidemic (027 ribotype) in a *C. difficile* infected individual. This approach has many applications in clinical setting where the rapid, accurate, pathogen identification is of critical importance. Such an approach could also be critical in the identification of biomarkers suitable for identification or stratification of those at risk to microbiota mediated disease.

Described below are examples of methods of the invention for identifying bacteria present in a sample, such as bacteria present in a sample which have/have not been cultured using a set of bacterial culture conditions, adjusting culture conditions as necessary to culture bacteria not cultured using an original set of bacterial culture conditions, obtaining whole genomic sequences and/or cultures of bacteria cultured using a suitable set of bacterial culture conditions.

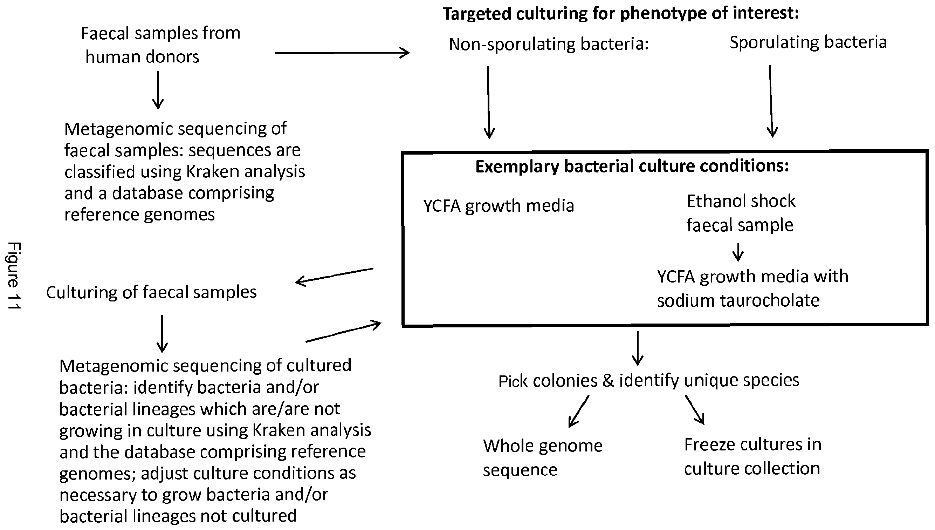

A schematic diagram of a work-flow encompassing the above methods is shown in Figure 11. The methods encompassed by the depicted work-flow can be performed separately, where applicable. For example, a method of identifying bacteria present in a sample as shown schematically on the left side of Figure 11 may be performed with or without performing an iteration of the culturing and metagenomics sequencing steps using alternate culture conditions.

### Example 3 - Analysis of the proportion of bacteria present in a sample which has been cultured

Faecal samples from 6 healthy humans were collected and the resident bacterial communities defined using a combined metagenomic sequencing and bacterial culturing approach using the complex, broad range culture medium, YCFA (Duncan et al., 2002). Applying shotgun metagenomic sequencing we profiled and compared the bacterial species present in the original faecal samples to those that grew on YCFA agar plates (by scraping the colonies off the plate for DNA isolation and sequencing). Importantly, we observed a strong correlation between the two (R² = 0.85) (Figure 12A) demonstrating that a significant proportion of the bacteria within the faecal microbiota can be cultured with a single growth medium. However, more than 8 × 10⁶ colonies would need to be picked and screened from agar plates to match the species detection sensitivity of metagenomic sequencing. Thus, we established a broad range culturing method that can be used to strategically identify novel bacteria with a defined phenotype.

The human intestinal microbiota is dominated by strict anaerobic bacteria that are extremely sensitive to ambient oxygen, so it is not known how these bacteria survive environmental exposure to transmit between individuals. Certain pathogenic Firmicutes, such as the diarrheal pathogen *Clostridium difficile,* produce metabolically dormant and highly resistant spores during colonization that facilitate both persistence within the host and environmental survival once shed (Francis et al., 2013; Janoir et al., 2013; Lawley et al., 2009). *C. difficile* spores have evolved mechanisms to resume metabolism and vegetative growth after intestinal colonisation by germinating in response to digestive bile acids (Francis et al., 2013). Relatively few intestinal spore-forming bacteria have been cultured to date and their genomes, phylogenies and phenotypes remain poorly characterised (Rajilic-Stojanovic et al., 2014). Recently, metagenomic studies have suggested that other unexpected members of the intestinal microbiota possess potential sporulation genes, even though these bacteria have never been grown in a laboratory and are not known to produce spores (Galperin et al., 2012; Abecasis et al., 2013; Meehan et al., 2014).

We hypothesized that sporulation is an unappreciated basic phenotype of the human intestinal microbiota that may have a profound impact on microbiota persistence and spread between humans. Spores from *C. difficile* are resistant to ethanol and this phenotype can be used to select for spores from a mixed population of spores and sensitive vegetative cells (Riley et al., 1987). Faecal samples were treated with ethanol and analysed using our combined culture and metagenomic approach. Principle component analysis demonstrated that ethanol treatment profoundly altered the culturable bacterial composition compared to the original profile and efficiently enriched for ethanol resistant bacteria, facilitating their isolation (Figure 12B). We next isolated individual bacterial colonies from faecal samples that grew on the YCFA medium with or without prior ethanol treatment and assigned them to a species or classified them as novel. We picked ~2000 bacterial colonies from both ethanol treated and ethanol non-treated conditions, re-streaked them to purity and performed full-length 16S rRNA gene sequencing. Sodium taurocholate can be added to the growth media to stimulate spore germination. The 16S rRNA gene sequences were compared to The Ribosomal Database Project (RDP) reference database to assign genus level taxonomy

(Wang et al., 2007). We imposed a BLASTn identity threshold of ≥98.7% over the full-length 16S rRNA gene sequence to define either a characterised or novel species (Clarridge et al., 2004; Bosshard et al., 2003; Altschul et al., 1990), and these were archived as frozen stocks for future analysis.

In total, we isolated bacteria from 97% of the most abundant genera and 90% of the most abundant species detected in our cohort by metagenomic sequencing. Even bacterial genera that were present at low relative abundance (<0.1 %) in the faecal samples were cultured. Overall, we cultured and archived 137 distinct bacterial species which included 45 novel species, and isolates representing 20 novel genera and 2 novel families. Our collection contains 90 species from the Human Microbiome Project's 'most wanted' list of previously uncultured and unsequenced microbes (Fodor et al., 2012). Thus, our broad-range culture approach led to massive bacterial discovery and challenges the notion that the majority of the intestinal microbiota is "unculturable".

By obtaining and then storing the genomic sequences of the bacterial isolates in a database, a database having more thorough genome coverage of intestinal microbiota, such as the HPMC database described in Annex A can be established.

### FINAL REMARKS

When used in this specification and claims, the terms "comprises" and "comprising", "including" and variations thereof mean that the specified features, steps or integers are included. The terms are not to be interpreted to exclude the possibility of other features, steps or integers being present.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting.

For the avoidance of any doubt, any theoretical explanations provided herein are provided for the purposes of improving the understanding of a reader. The inventors do not wish to be bound by any of these theoretical explanations.

### REFERENCES

All references referred to herein are hereby incorporated by reference.
Abecasis, A. B. et al. A genomic signature and the identification of new sporulation genes. Journal of bacteriology 195, 2101-2115, doi:10.1128/JB.02110-12 (2013).
Altschul, S. F., Gish, W., Miller, W., Myers, E. W. & Lipman, D. J. Basic local alignment search tool. Journal of molecular biology 215, 403-410, doi:10.1016/S0022-2836(05)80360-2 (1990).
Bosshard, P. P., Abels, S., Zbinden, R., Bottger, E. C. & Altwegg, M. Ribosomal DNA sequencing for identification of aerobic gram-positive rods in the clinical laboratory (an 18-month evaluation). J Clin Microbiol 41, 4134-4140 (2003).
Clarridge, J. E., 3rd. Impact of 16S rRNA gene sequence analysis for identification of bacteria on clinical microbiology and infectious diseases. Clinical microbiology reviews 17, 840-862, table of contents, doi:10.1128/CMR.17.4.840-862.2004 (2004).
Duncan, S. H., Hold, G. L., Harmsen, H. J., Stewart, C. S. & Flint, H. J. Growth requirements and fermentation products of Fusobacterium prausnitzii, and a proposal to reclassify it as Faecalibacterium prausnitzii gen. nov., comb. nov. Int J Syst Evol Microbiol 52, 2141-2146 (2002).
Fodor, A. A. et al. The "most wanted" taxa from the human microbiome for whole genome sequencing. PloS one 7, e41294, doi:10.1371/journal.pone.0041294 (2012).
Francis, M. B., Allen, C. A., Shrestha, R. & Sorg, J. A. Bile acid recognition by the Clostridium difficile germinant receptor, CspC, is important for establishing infection. PLoS pathogens 9, e1003356, doi:10.1371/journal.ppat.1003356 (2013).
Galperin, M. Y. et al. Genomic determinants of sporulation in Bacilli and Clostridia: towards the minimal set of sporulation-specific genes. Environmental microbiology 14, 2870-2890, doi:10.1111/j.1462-2920.2012.02841.x (2012).
Goodman et al., PNAS, vol. 108, 6252-6257 (2011)
Janoir, C. et al. Adaptive strategies and pathogenesis of Clostridium difficile from in vivo transcriptomics. Infect Immun 81, 3757-3769, doi:10.1128/IAI.00515-13 (2013).
Lawley, T. D. and A. W. Walker (2013). "Intestinal colonization resistance." Immunology 138(1): 1-11.
Lawley, T. D. et al. Antibiotic treatment of clostridium difficile carrier mice triggers a supershedder state, spore-mediated transmission, and severe disease in immunocompromised hosts. Infect Immun 77, 3661-3669, doi:10.1128/IAI.00558-09 (2009).
Meehan, C. J. & Beiko, R. G. A phylogenomic view of ecological specialization in the Lachnospiraceae, a family of digestive tract-associated bacteria. Genome biology and evolution 6, 703-713, doi:10.1093/gbe/evu050 (2014).
Petrof, E. O., G. B. Gloor, S. J. Vanner, S. J. Weese, D. Carter, M. C. Daigneault, E. M. Brown, K. Schroeter and E. Allen-Vercoe (2013). "Stool substitute transplant therapy for the eradication of Clostridium difficile infection: 'RePOOPulating' the gut." Microbiome 1(1): 3.
Rajilic-Stojanovic, M. & de Vos, W. M. The first 1000 cultured species of the human gastrointestinal microbiota. FEMS microbiology reviews 38, 996-1047, doi:10.1111/1574-6976.12075 (2014).
Riley, T. V., Brazier, J. S., Hassan, H., Williams, K. & Phillips, K. D. Comparison of alcohol shock enrichment and selective enrichment for the isolation of Clostridium difficile. Epidemiology and infection 99, 355-359 (1987).
Seekatz, A. M., J. Aas, C. E. Gessert, T. A. Rubin, D. M. Saman, J. S. Bakken and V. B. Young (2014). "Recovery of the gut microbiome following fecal microbiota transplantation." MBio 5(3): e00893-00814.
Stewart, E. J. (2012). "Growing unculturable bacteria." J Bacteriol 194(16): 4151-4160.
van Nood, E., A. Vrieze, M. Nieuwdorp, S. Fuentes, E. G. Zoetendal, W. M. de Vos, C. E. Visser, E. J. Kuijper, J. F. Bartelsman, J. G. Tijssen, P. Speelman, M. G. Dijkgraaf and J. J. Keller (2013). "Duodenal infusion of donor feces for recurrent Clostridium difficile." N Engl J Med 368(5): 407-415.
Wang, Q., Garrity, G. M., Tiedje, J. M. & Cole, J. R. Naive Bayesian classifier for rapid assignment of rRNA sequences into the new bacterial taxonomy. Applied and environmental microbiology 73, 5261-5267, doi:10.1128/AEM.00062-07 (2007).

### ANNEX A - DESCRIPTION OF THE HUMAN PAN-MICROBE COMMUNITIES (HPMC) DATABASE

### ABSTRACT

The Human Pan-Microbe Communities (HPMC) database (http://www.hpmcd.org) provides a manually curated, searchable, metagenomic resource to facilitate investigation of human gastrointestinal microbiota. Over the past decade, the application of metagenome sequencing to elucidate the microbial composition and functional capacity present in the human microbiome has revolutionized many concepts in our basic biology. When sufficient high quality reference genomes are available, whole genome metagenomic sequencing of microbiota samples can provide direct biological insights and high-resolution phylogenetic classification. The HPMC database provides species level, standardized phylogenetic classification of over 1800 human gastrointestinal whole genome metagenomic samples. This is achieved by combining culture derived bacterial genomes and over 21000 public reference genomes representing bacteria, viruses, archaea and fungi with manually curated species classification and enhanced sample metadata annotation. A user-friendly, web based interface provides the ability to search for (1) microbial groups associated with health or disease state, (2) the health or disease states associated with presence of microbial group, (3) the enrichment of a microbial gene or sequence and (4) enrichment of a functional annotation. The HPMC database enables detailed analysis of human metagenomic communities and supports research from basic microbiology and immunology to biomarker and therapeutic development in human health and disease.

### INTRODUCTION

The importance of microbial communities and the complex functional roles they perform in human health and disease is becoming increasingly evident (1-4). Large-scale projects such as the Human Microbiome Project and MetaHIT, have provided considerable advancements in this area of research (1,2). The growing availability of metagenomic sequencing and associated analysis tools has enabled quantification and understanding of this microbial diversity at a level not previously possible. While traditional 16S rRNA based profiling approaches were limited to bacteria and archaea, whole genome sequence or shotgun metagenomics, expands analysis to eukaryotic, fungal and DNA based viruses that do not possess a 16S rRNA gene. Coupled with computational analysis and high quality reference genomes this enables higher phylogenetic resolution (e.g. species or even strain level relationships), where no differences would be detectable using only 16S rRNA gene profiling.

The importance of understanding the tremendous molecular and phenotypic diversity present within single bacterial "species" or lineage has only recently become evident. Whole genome phylogeny of hundreds of isolates from a single "species" has demonstrated each "species" represents an evolutionary web of highly related lineages with diverse phenotypic characteristics (5-8). For example, strains of many opportunistic pathogen species including *Peptoclostridium difficile* and *Escherichia coli,* that cannot be differentiated by 16S rRNA gene profiling, can range from benign members of the gastrointestinal tract to highly virulent pathogens, inducing severe, sometimes fatal symptoms in the host (5,9,10). Though less well studied, similarly important phylogenetic relationships are also likely to occur in fungal and viral species.

While many excellent repositories for metagenomic sequence datasets already exist, these tools serve primarily to support wide-scale submission, data archive and generic analysis functions suitable for microbiota across many environments (11-14). In the human gastrointestinal tract, focused research effort has resulted in sufficient sampling, underlying biological knowledge and high quality reference genomes to enable specialized analysis approaches. The microbiota of the human gastrointestinal tract represents approximately 70% of all human microbiota, and plays a critical role in our sustenance, immune system development and protection against infection. In this important area of research, the ability to incorporate a comprehensive measure of microbial diversity with high phylogenetic resolution will facilitate the progression from current basic, correlation-based observational studies to microbe identification and experimental validation of causative relationships. In human health, this capacity will provide insights from basic biology and disease understanding to identification of biologically relevant biomarkers and ultimately inform targeted therapeutic intervention (15,16).

We present the Human Microbial Communities (HPMC) database, a database of human gastrointestinal microbiota derived from faecal samples. The HPMC database currently incorporates 4425 whole genome metagenomics sequencing runs, derived from 1830 independent samples (approximately 41% of all public EBI-metagenomics portal samples (11) and 29% of all MG-RAST public whole genome metagenomic samples (12)). These samples were subjected to stringent quality control and a standardized, specifically designed analysis process described in detail below. This process leverages an optimized, manually curated list of over 21000 genomes for sequence read classification. Classification in this manner provides species and the potential for strain level resolution and also includes other important viral, fungal and eukaryotic members of the microbiota community that are undetectable with 16S rRNA gene profiling. In addition, the raw metagenomic sequences are utilized to support extensive functional analysis and sequence-based search functionality. This enhanced classification and comprehensive sequence analysis is supplemented with manually curated metadata to facilitate complex, multidimensional sample filtering and search queries across currently disparate datasets at the sample, species, functional and sequence level.

### DATA SOURCES AND PROCESSING

The HPMC database represents a highly specialized, human sample specific extension to the standard EBI metagenomics portal (EMP). Sample data are integrated from high quality metagenomics datasets within the EMP that are enhanced with manually curated sample metadata and a culture derived, comprehensive genome collection for phylogenetic analysis.

All raw reads obtained from whole genome metagenomic sequencing of human faecal samples are considered for inclusion, however, samples with low read coverage or poor read quality resulting in loss of greater than 25% of reads are excluded from further analysis at this stage and not included in HPMC database. Samples with insufficient metadata to identify health or disease state are also excluded at this stage. Publicly available metagenomic samples that are available in the nucleotide archives but absent in the EMP and pass these quality criteria have also been included. Reads from included samples undergo quality filtering with Trimommatic v0.33 (17), high quality gene fragments are identified using FragGeneScan v1.19 (18) and functional annotation performed using InterProScan v5.0 (19) as described previously (11). Identified gene fragments are also included in a reference BLAST database to provide sequence similarity search functionality (20).

One of the fundamental features of the HPMC database lies in the ability to provide detailed taxonomic resolution for gastrointestinal microbes. The Kraken algorithm (21) provides the ability to classify whole genome metagenomic reads based on a k-mer lowest common ancestor database generated from whole genome sequences. The HPMC analysis process applies the Kraken v0.10.6 approach to classify reads against a custom database populated with complete bacterial, archaeal, fungal and viral genomes and 216 bacterial genomes derived from bacterial cultures isolated directly from human faecal samples.

Knowledge of the gastrointestinal tract specifically is incorporated within the HPMC to generate a "gold-standard," manually curated list of species that have been experimentally cultured from faecal samples. In addition, it is possible for users to register for a free account on HPMC database. Registration enables users to independently annotate species as known members of the gastrointestinal microbiota. These annotations are saved to the users personal account and contribute to the overall, community-wide, classification of gastrointestinal species available for searching and filtering by all users of the HPMC database.

Quantification and comparison of metagenomic samples is dependent on accurate normalization between phylogenetic groups, samples and experiments. Variability in the availability of genomes from different phylogenetic groups impacts the classification potential when applying the lowest common ancestor approach. For example, while classification to the species level when only a few representative species are described within a genus can be readily achieved, accurate species classification is unlikely due to poor representation across species diversity. Inclusion of many species, such as employed in the HPMC database increases the percentage of reads that are only able to be classified at the genus level due to extensive gene homology. It is therefore necessary to correct for this bias prior to performing conventional transformations, standardization and sample scaling. The HPMC overcomes this limitation by correcting assigned read counts at the phylogenetic level by genome uniqueness. Here, genome uniqueness is defined as the percentage of the genome where a 100 bp sliding window would uniquely identify that genome amongst all genomes contained in the database. This approach corrects for uneven genome coverage across phylogenetic group enabling direct comparison between species. The resulting corrected counts are subjected to log transformation, samples standardized to a mean of 0 and multiple sample scaling performed according to best practice metagenomic data analysis (12). This approach provides the ability to perform comprehensive metagenomic analysis across diverse phylogenetic groups with variable genome representation.

To facilitate complex, multi-faceted search functionality and supplement incomplete and poorly annotated metagenomics samples, manual searching of the original published articles were performed for each study included in the HPMC database to supplement metadata and capture the maximum information available for each sample. This approach supplements the minimal data required for submission to public repositories and updates metadata associated with older sample submissions to reflect current standards. The results of metagenomic sample analysis and functional annotation were combined with this manually curated metadata into a relational database designed to support complex querying and advanced analysis functionality.

### ANALYSIS CAPABILITY

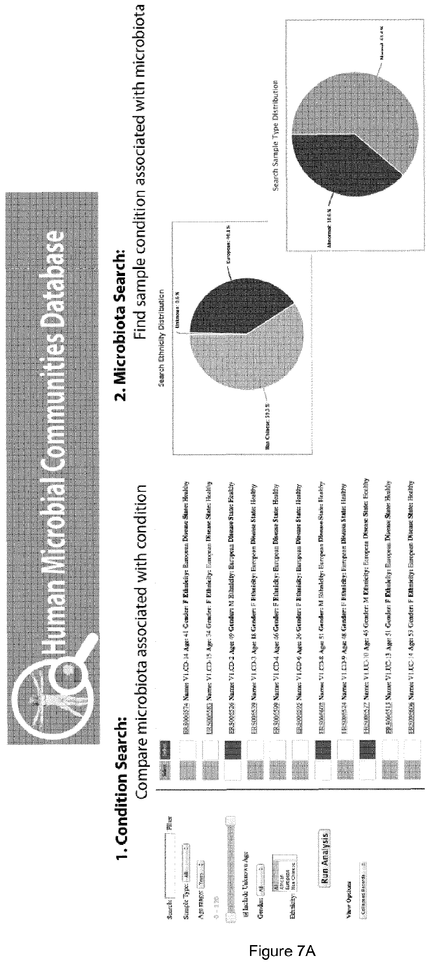

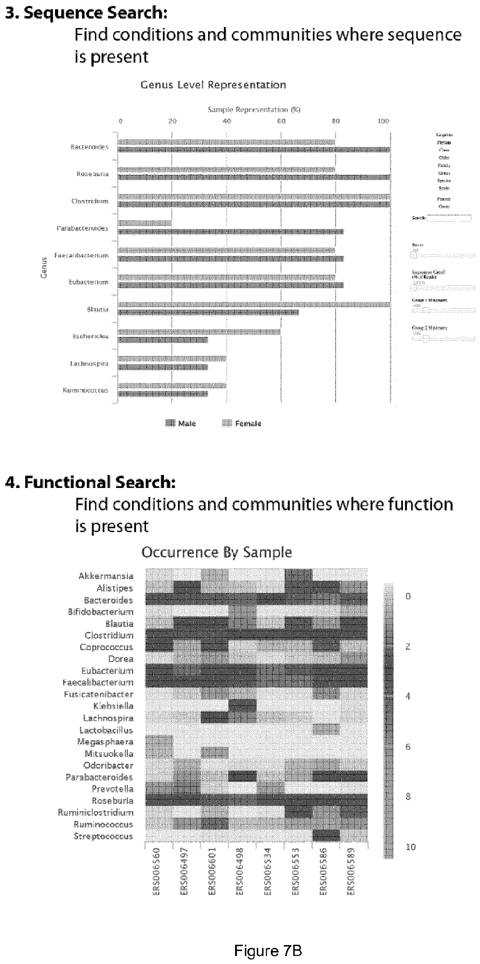

The HPMC database is designed to support 4 types of searches. These are to: 1) search by sample facet (i.e. human health or disease state) to identify microbiota compositions or specific microbial groups, 2) search by microbiota composition or specific microbial groups to identify sample type and community correlations, 3) search by sequence (i.e. microbiota or other gene sequence) or 4) search by functional annotation to identify associated sample type and microbial groups (Fig. 7).

### 1. Search for microbial groups associated with health or disease state

The sample search function is designed for users with knowledge of the host biology and interest in the microbiota that correlates with health or disease state. The search interface allows the user to perform detailed metadata based sample filtering by parameters including health status, age, gender, geography and ethnicity. Samples can be allocated to one of two user-defined groups with comparisons performed between the microbiota communities in each condition. For example, one may wish to compare the microbiota detected in samples with a particular disease state such as Inflammatory Bowel Disease (IBD) with all other healthy samples in the database. On performing this analysis a clear decrease is observed in the representation of Firmicutes and Bacteroidetes in the community associated with the disease state. At the species level, analysis suggests a loss of *Faecalibacterium prausnitzii, Bacteroides vulgatus* and two species of the Roseburia genus (*R. hominis and R. faecis*). This result is consistent with the core EMP that suggests a decrease in one or more of the 193 known Ruminococcaceae and one or more of the 713 known Lachnospiraceae family members. These results demonstrate the improved resolutions provided by the HPMC with the loss of both *F. prausnitzii* and Roseburia species previously reported to be associated with IBD (). Functionality is also provided to filter this search to include only samples obtained from a particular ethnicity, gender or age profile. The user interface provides full flexibility to this search approach, supporting the comparison of any two groups of samples filtered by any combination of search parameters.

### 2. Search for health or disease states associated with presence of microbial group

While the majority of existing metagenomic analysis focuses on identification of the microbial compositions within particular samples, the HPMC database also provides the ability to search by a component of the microbiota. For example, one may be interested to identify the sample conditions and microbial community structure where *Helicobacter pylori,* a common risk factor for gastric cancer, exists in healthy individuals. The search functionality provided by the HPMC database enables identification of the samples where a microbial component or phylogenetic group, such as *H. pylori,* is represented at a level higher or lower than is observed across the complete population of samples within the database. This analysis is particularly powerful for researchers with expertise in specific microbes and provides the ability to identify previously unknown conditions in which the microbes are dominant. Analysis of co-occurring species and community structure may also be applied to provide functional insights into health associated microbiota communities and assist in the prediction of host interactions and responses.

### 3. Search by enrichment of a microbial gene or sequence

Expanding on the benefits of whole genome metagenomic sequencing over traditional 16S rRNA gene profiling, the HPMC database provides the ability to search for microbial genes or sequences of interest directly detected within the sample. Each sample is characterized by the presence, defined as detection above a user-defined percentage similarity, of a particular searched sequence. The samples in which the searched sequence is detected are compared to the remaining samples within the database where the sequence was not found to be present at the defined homology cut-off. The sequence search functionality enables users to search independently of known, curated functional information. For example if one discovers a novel antibiotic resistance gene, the HPMC database provides the ability to determine the conditions in which species possessing this gene are detected and identify common microbiota community structures and sample conditions associated with this gene of interest.

### 4. Search by enrichment of a functional annotation

To complement the sequence homology searching, the HPMC database also provides the ability to search for samples with specific functional annotations. This search functionality enables detailed identification of sample types or community structures typically associated with a particular biological function. As described for sequence homology based searches, samples will be compared based on presence or absence of the defined functional annotation. This approach enables the discovery of sample conditions and microbiota community structure specific to the functional annotation, such as virulence, metabolism, sporulation and immune system evasion. Knowledge of the conditions where functional capacity is present or absent is fundamental to the development of the basic biological understanding needed to further microbiota research.

### DATA AVAILABILITY

To ensure compatibility with existing resources, standardized ERS/SRS identifiers are used to identify raw reads and NCBI identifiers are used for genome identification. Direct links are provided to the relevant records on these sites where appropriate. Analysis results are also provided for export as a single download file to support further investigation. Throughout all search results, images are available for export in PDF, PNG, JPG and SVG formats. Complete raw data for the entire database is available as a flat-file download from the help pages (http://www.hpmcd.org/help.php).

### FUTURE DEVELOPMENTS

The close association between the HPMC database and the EMP ensures continued inclusion of newly published, relevant human metagenomic studies as they become publicly available. This important linkage ensures that the HPMC database will continue to provide access to the most comprehensive selection of human gastrointestinal metagenomic datasets complemented by a specialized analysis process and detailed manual curation.

The sophisticated analysis framework will continue to be expanded, providing additional search functionality consistent with the standard metadata requirements incorporated in the data submission process. Improvements in the complexity and specificity of search functionality will also become possible as the diversity of samples increases to include further, diverse infections, disease conditions and sample types.

As the user base continues to expand the comprehensive nature of the "cultured" and community annotated gastrointestinal species annotation will also increase. Over time this resource will grow to become a unique, community consensus of human gastrointestinal bacteria, providing an extra level of annotation for analysis of metagenomic datasets. In parallel, as the diversity of complete reference genomes from cultured species expands and the number of whole genome metagenomic experiments increases, the scope of the HPMC database will also be widened from the current focus on human gastrointestinal tract to encompass samples from other human body sites including female reproductive tract, lungs and bladder within the same computational framework.

### CONCLUSIONS

The HPMC expands on the general-purpose metagenomic analysis capabilities provided by the EMP to provide a tailored analysis platform capable of supporting the specific, search requirements for human metagenomic data and medical research. As the use of whole genome metagenomic sequencing expands and the number of high quality reference genomes increases, the ability to perform integrated analysis of samples from multiple independent studies becomes increasingly necessary. While many individual studies may suffer from limited statistical power due to small sample size, integrated meta-analysis as provided in the HPMC database overcomes many of these limitations. In this context the HPMC database represents the next step in metagenomic analysis, supporting the progression from generic sample archive and correlation studies to biologically relevant candidate identification suitable for direct experimental validation and human medical applications.

### TABLE AND FIGURES

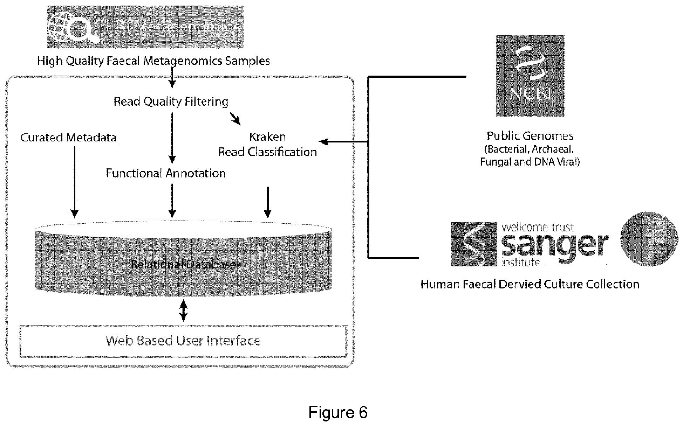

### Fig. 6: Overview of HPMC database structure and analysis process

High quality sequences from human faecal metagenomic samples are combined with manually curated and sample metadata. Phylogenetic classification is performed in the context of over 21000 curated genomes. Sample and functional annotation data are stored in a relational database and available for querying through a freely available web based interface.

### Fig. 7: Condition and Microbiota Search Functionality

The search functionality provides the ability to query by (1) samples (filtered by manually curated metadata), (2) specific microbes (3) sequence similarity or (4) functional annotation. Summary data about the identified samples and microbiota composition are provided as raw data, bar charts, heat maps and pie charts.

### REFERENCES

1. Human Microbiome Project, C. (2012) Structure, function and diversity of the healthy human microbiome. Nature, 486, 207-214.
2. Qin, J., Li, R., Raes, J., Arumugam, M., Burgdorf, K.S., Manichanh, C., Nielsen, T., Pons, N., Levenez, F., Yamada, T. et al. (2010) A human gut microbial gene catalogue established by metagenomic sequencing. Nature, 464, 59-65.
3. Turnbaugh, P.J., Hamady, M., Yatsunenko, T., Cantarel, B.L., Duncan, A., Ley, R.E., Sogin, M.L., Jones, W.J., Roe, B.A., Affourtit, J.P. et al. (2009) A core gut microbiome in obese and lean twins. Nature, 457, 480-484.
4. Hsiao, A., Ahmed, A.M., Subramanian, S., Griffin, N.W., Drewry, L.L., Petri, W.A., Jr., Haque, R., Ahmed, T. and Gordon, J.I. (2014) Members of the human gut microbiota involved in recovery from Vibrio cholerae infection. Nature, 515, 423-426.
5. He, M., Sebaihia, M., Lawley, T.D., Stabler, R.A., Dawson, L.F., Martin, M.J., Holt, K.E., Seth-Smith, H.M., Quail, M.A., Rance, R. et al. (2010) Evolutionary dynamics of Clostridium difficile over short and long time scales. Proceedings of the National Academy of Sciences of the United States of America, 107, 7527-7532.
6. Okoro, C.K., Kingsley, R.A., Connor, T.R., Harris, S.R., Parry, C.M., Al-Mashhadani, M.N., Kariuki, S., Msefula, C.L., Gordon, M.A., de Pinna, E. et al. (2012) Intracontinental spread of human invasive Salmonella Typhimurium pathovariants in sub-Saharan Africa. Nature genetics, 44, 1215-1221.
7. Holt, K.E., Baker, S., Weill, F.X., Holmes, E.C., Kitchen, A., Yu, J., Sangal, V., Brown, D.J., Coia, J.E., Kim, D.W. et al. (2012) Shigella sonnei genome sequencing and phylogenetic analysis indicate recent global dissemination from Europe. Nature genetics, 44, 1056-1059.
8. Mutreja, A., Kim, D.W., Thomson, N.R., Connor, T.R., Lee, J.H., Kariuki, S., Croucher, N.J., Choi, S.Y., Harris, S.R., Lebens, M. et al. (2011) Evidence for several waves of global transmission in the seventh cholera pandemic. Nature, 477, 462-465.
9. Picard, B., Garcia, J.S., Gouriou, S., Duriez, P., Brahimi, N., Bingen, E., Elion, J. and Denamur, E. (1999) The link between phylogeny and virulence in Escherichia coli extraintestinal infection. Infection and immunity, 67, 546-553.
10. He, M., Miyajima, F., Roberts, P., Ellison, L., Pickard, D.J., Martin, M.J., Connor, T.R., Harris, S.R., Fairley, D., Bamford, K.B. et al. (2013) Emergence and global spread of epidemic healthcare-associated Clostridium difficile. Nature genetics, 45, 109-113.
11. Hunter, S., Corbett, M., Denise, H., Fraser, M., Gonzalez-Beltran, A., Hunter, C., Jones, P., Leinonen, R., McAnulla, C., Maguire, E. et al. (2014) EBI metagenomics--a new resource for the analysis and archiving of metagenomic data. Nucleic acids research, 42, D600-606.
12. Meyer, F., Paarmann, D., D'Souza, M., Olson, R., Glass, E.M., Kubal, M., Paczian, T., Rodriguez, A., Stevens, R., Wilke, A. et al. (2008) The metagenomics RAST server - a public resource for the automatic phylogenetic and functional analysis of metagenomes. BMC bioinformatics, 9, 386.
13. Sharma, V.K., Kumar, N., Prakash, T. and Taylor, T.D. (2010) MetaBioME: a database to explore commercially useful enzymes in metagenomic datasets. Nucleic acids research, 38, D468-472.
14. Markowitz, V.M., Chen, I.M., Palaniappan, K., Chu, K., Szeto, E., Pillay, M., Ratner, A., Huang, J., Woyke, T., Huntemann, M. et al. (2014) IMG 4 version of the integrated microbial genomes comparative analysis system. Nucleic acids research, 42, D560-567.
15. Forster, S.C. and Lawley, T.D. (2015) Systematic discovery of probiotics. Nature biotechnology, 33, 47-49.
16. Adamu, B.O. and Lawley, T.D. (2013) Bacteriotherapy for the treatment of intestinal dysbiosis caused by Clostridium difficile infection. Current opinion in microbiology, 16, 596-601.
17. Bolger, A.M., Lohse, M. and Usadel, B. (2014) Trimmomatic: a flexible trimmer for Illumina sequence data. Bioinformatics, 30, 2114-2120.
18. Rho, M., Tang, H. and Ye, Y. (2010) FragGeneScan: predicting genes in short and error-prone reads. Nucleic acids research, 38, e191.
19. Jones, P., Binns, D., Chang, H.Y., Fraser, M., Li, W., McAnulla, C., McWilliam, H., Maslen, J., Mitchell, A., Nuka, G. et al. (2014) InterProScan 5: genome-scale protein function classification. Bioinformatics, 30, 1236-1240.
20. Camacho, C., Coulouris, G., Avagyan, V., Ma, N., Papadopoulos, J., Bealer, K. and Madden, T.L. (2009) BLAST+: architecture and applications. BMC bioinformatics, 10, 421.
21. Wood, D.E. and Salzberg, S.L. (2014) Kraken: ultrafast metagenomic sequence classification using exact alignments. GENOME Biology, 15, R46.

### ANNEX B - EXAMPLE WORKFLOW FOR UTILISING THE HPMC DATABASE

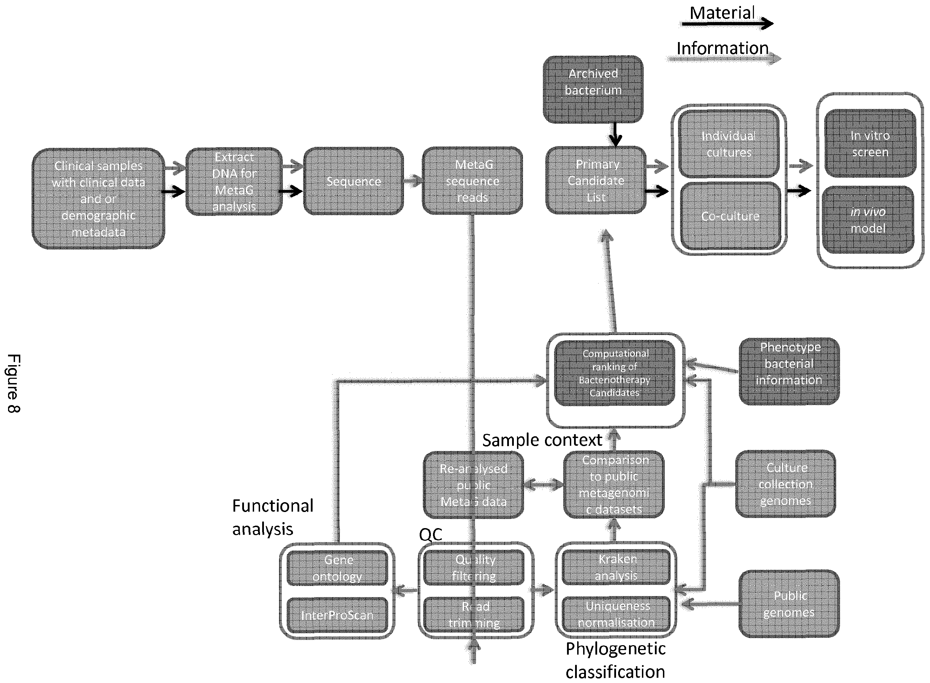

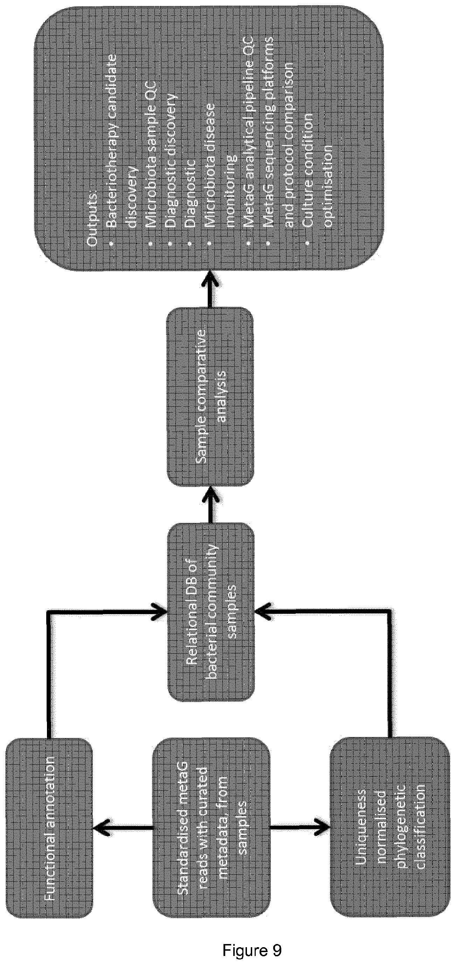

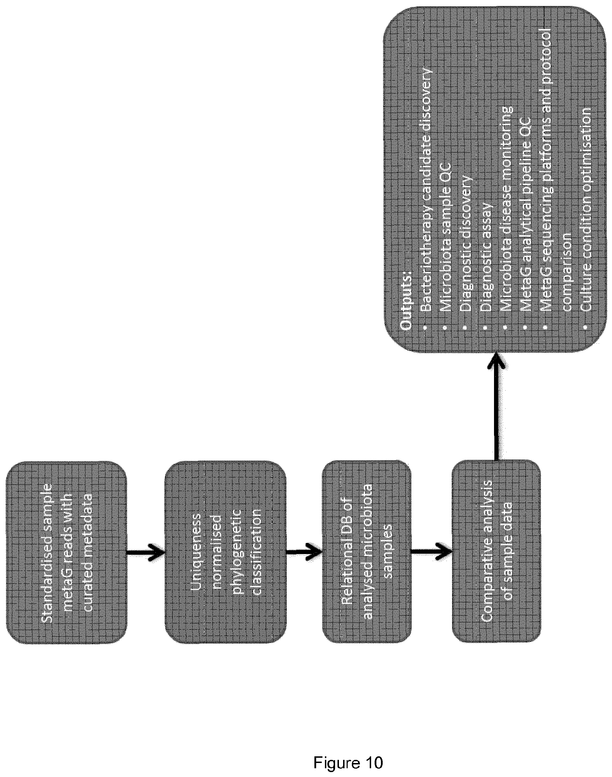

Fig. 8 illustrates an example workflow for utilising the HPMC database as well as the techniques described above. Fig. 9 and Fig. 10 provide further details regarding this workflow.

The aspects of the workflow shown in Fig. 8 can be better understood with reference to the following discussion:

### Clinical samples with clinical data and or demographic metadata

Could consist of faecal samples from patients suffering from Clostridium difficile infection and also patients with autoimmune diseases such as IBD.

### Extract DNA for MetaG analysis

DNA can be extracted from sample for sequencing, we use FastDNA SPIN Kit for soil. This is a standard procedure

### Sequence

Sequencing is carried out on Illumina Hi-Seq 125bp read length. This is a standard procedure.

### MetaG sequence reads

Output from sequencing. These are then input to metagenomic analysis

### Archived bacterium

Consists of phenotyped culture collection. The culture collection is currently not in public domain. Novelty is derived from novel uncharacterised bacteria in culture collection.

### Primary candidate list

Using the information from the metagenomic analysis primary candidates can be picked from the culture collection.

### Individual cultures/co-culture

Primary candidate mixtures can consist of single or multiple candidates.

### In vitro screen

Test efficacy of candidates against C. difficile by growth competition assays in fermentation system or on agar plates. Also important is combination of cultures and their ability to grow with each other.

### In vivo model

Inoculate Germ Free Mice (GFM) with human microbiota. This provides a model to challenge with pathogens and then to resolve using a bacteriotheraphy. Humanised mouse model is ideal scenario, we also have mouse microbiota model if necessary.

### Quality Filtering

Reads are filtered according to standard best practice low quality bases from the end of the read are removed and low quality reads removed entirely.

### Read Trimming

Adapter sequences are removed from the ends of reads using Trimmomatic.

### Gene Ontology/lnterproScan

Gene ontology annotation and InterProScan5.0 annotation is applied to raw reads identified through FragGeneScan to provide functional annotation of genes within metagenomic sample

### Kraken Analysis

Reads are assigned to the lowest taxonomic classification using a lowest common ancestor approach. This implements the Kraken algorithm with a custom database based on the culture collection genomes and public high quality genomes

### Uniqueness Normalization

Normalization of reads to account for read coverage and species bias in phylogenetic representation. Provides the ability to accurately detect and compare between species and strains across variable phylogenetic context, genome availability and sequence depth.

### Comparison to Public Metagenomic Datasets

Analysis of diseased and healthy samples and in the context of public datasets from other healthy and diseased individuals provides the ability to identify key species or community structure associated with the disease of interest.

### Re-analysed public MetaG datasets

Iterative process involving re-analysis of new metagenomic data.

### Computational ranking of Bacteriotherapy Candidates

Isolates of species identified as different between healthy and diseased states are selected from the culture collection. The genomes are screened for antibiotic and virulence markers to eliminate species not suitable for bacteriotherapy.

The results from functional analysis of healthy and diseased states is utilized to identify critical functions.

Prioritised ranking would be further filtered by phylogenetic relationships and ease of growing the isolate in question.

Species that contain these functions will be prioritized as candidates

### Phenotype bacterial information

Phenotypic information for bacterial candidates is included in the computational ranking of bacteriotherapy candidates . Phenotyping is incorporated into archived bacterium box.

### Culture collection genomes

Provide reference sequence to map metagenomic reads against. Also act as input for computational bacteriotherapy candidates.

### Public genomes

Act as input to the metagenomic database- healthy and diseased data-sets can be included.

## Claims

1. A method of analysing microorganisms present in a sample using a database that stores a plurality of microbial reference genomes for the microorganisms and phylogenetic information which relates the stored reference genomes to each other in a phylogenetic structure, the method including:
using a plurality of sequence reads obtained from a sample to count the number of sequence reads deemed to uniquely map to each of a plurality of reference genomes within the phylogenetic structure by;
for the/each reference genome in the plurality of reference genomes:
comparing the plurality of sequence reads with the reference genome to establish whether any sequence reads map to the reference genome and to no other reference genome stored in the database;
if a sequence read maps to the reference genome and to no other reference genome stored in the database, counting the sequence read as being deemed to uniquely map to the reference genome; and
for each of the plurality of reference genomes to which at least one sequence read has been deemed uniquely mapped, normalizing the number of sequence reads counted as being deemed uniquely mapped to the reference genome by dividing the counted number of sequence reads by a measure that reflects the uniqueness of the reference genome and comparing the normalized number of sequence reads to the normalized number of sequence reads for the other reference genomes so as to obtain an indication of the relative abundance of the microbial reference genomes of the microorganisms within the sample,
wherein the measure that reflects the uniqueness of the reference genome is determined by:
for the/each reference genome:
identifying one or more genetic sequences deemed to uniquely identify the reference genome; and
determining the measure that reflects the uniqueness of the reference genome by dividing the combined length of the one or more genetic sequences deemed to uniquely identify the reference genome by the length of the corresponding reference genome, and
wherein identifying one or more genetic sequences deemed to uniquely identify each of the plurality of reference genomes includes,
for each reference genome in the database:
defining a plurality of segments, each segment containing a different genetic sequence from the reference genome;
for the genetic sequence contained in each segment:
comparing the genetic sequence contained in the segment with the entirety of the genetic content of all other reference genomes in the database to establish whether the segment maps to any of the other reference genomes, wherein establishing whether the segment maps to any of the other reference genomes ignores minor differences of two to three base pairs for a segment which is 100 base pairs in length; and
if the genetic sequence contained in the segment maps to no other reference genome in the database, identifying the genetic sequence contained in the segment as being deemed to uniquely identify the reference genome.

2. The method according to claim 1, wherein the plurality of segments defined for each reference genome have a predetermined length, and include each possible segment of that length that could be defined for the reference genome.

3. The method according to any previous claim, wherein the database includes an entry for each reference genome within the phylogenetic structure, and preferably the entry for each reference genome includes a parent field for storing a pointer to a parent of the reference genome within the phylogenetic structure.

4. The method according to claim 3, wherein the entry for each reference genome includes a uniqueness field for storing the measure that reflects the uniqueness of the reference genome.

5. The method according to claim 4, wherein the uniqueness field is recalculated each time a new reference genome is stored in the database.

6. The method according to any previous claim, wherein the sequence reads are obtained by a shotgun sequencing process in which the DNA contained in the sample is broken up randomly into small segments which are then sequenced to obtain the plurality of sequence reads, wherein the plurality of sequence reads from the sample are obtained from across the complete DNA of organisms within the sample.

7. The method according to any previous claim, wherein the sequence reads each have a length of at least 80 or more base pairs.

8. The method of any previous claim including:
obtaining a plurality of sequence reads from a first portion of the sample and obtaining indications of the relative abundances of one or more microorganisms within the first portion of the sample;
obtaining a plurality of sequence reads from microorganisms cultured from a second portion of the sample using a microbial culturing method and obtaining indications of the relative abundances of one or more microorganisms within the cultured portion of the sample; and
comparing the indications of the relative abundances of the microorganisms within the first portion of the sample with the indications of the relative abundance of the microorganisms within the second portion of the sample.

9. The method according to claim 8, further comprising:
obtaining a plurality of sequence reads from microorganisms cultured from a third portion of the sample, obtained from the same source as the first and second portions of the samples using an alternate microbial culturing method and obtaining indications of the relative abundances of one or more microorganisms within the third portion of the sample; and
comparing the indications of the relative abundances of the microorganisms within the third portion of the sample with the indications of the relative abundance of the microorganisms within at least one of the first and second portions of the samples.

10. The method according to claim 8, including:
determining a microorganism of interest present in the sample which were cultured using the microbial culturing method by comparing the indications of the relative abundances of the microorganisms within the first portion of sample with the indications of the relative abundance of the microorganisms within the second portion of the sample;
employing the microbial culturing method to prepare cultures of one or more of the microorganisms of interest from the sample, and
determining the genomic sequence(s) of said microorganism.

11. The method according to claim 9, further comprising adding the genomic sequence(s) of said one or more of the microorganisms of interest to the database that stores reference genomes.

12. The method according to any one of claims 1 to 11, wherein the sample is obtained from a human.

13. The method according to any one of claims 1 to 12, further comprising using the database to correlate the obtained relative abundance of the microorganisms within the sample with health or disease state of the host from which the sample was obtained by comparing the obtained relative abundance of the microorganisms in samples with a particular disease with healthy samples in the database.

14. The method according to any one of claims 1 to 13, wherein the microorganism is a bacterium.

15. A method of identifying one or more bacteria for bacteriotherapy for a dysbiosis or a disease, the method comprising:
obtaining a plurality of sequence reads from a sample obtained from a patient with the dysbiosis or the disease;
using a method according to any one of claims 1 to 14 to obtain indications of the relative abundances of bacteria within the sample;
comparing the indications of the relative abundances of the bacteria within the sample with the relative abundance of the bacteria in a control;
selecting one or more bacteria with a genome, which is absent from the sample obtained from the patient but present in the control, or which is present at a lower relative abundance in the sample obtained from the patient compared with the control, for bacteriotherapy for the dysbiosis or the disease.

16. A method of identifying one or more bacteria for bacteriotherapy for a dysbiosis or a disease, the method comprising:
obtaining a plurality of sequence reads from (i) a first sample obtained from a patient with the dysbiosis or the disease;
obtaining a plurality of sequence reads from (ii) a second sample obtained from the same patient after the patient has received a faecal transplant;
using a method according to any one of claims 1 to 14 to obtain indications of the relative abundances of bacteria within the first and second samples;
comparing the indications of the relative abundances of the bacteria within the first sample with the relative abundance of the bacteria in the second sample;
selecting one or more bacteria with a genome, which is absent from the first sample but present in the second sample, or which is present at a lower relative abundance in the first sample compared with the second sample, for bacteriotherapy for the dysbiosis or the disease.

17. A method of identifying one or more bacteria for therapy of a disease which is **characterised by** the presence of a pathogenic bacterium but for which no disease symptoms are exhibited by an asymptomatic carrier of the pathogenic bacterium, the method comprising:
obtaining a plurality of sequence reads from a first sample obtained from an asymptomatic carrier of the pathogenic bacterium and obtaining a plurality of sequence reads from a second sample obtained from a healthy individual;
using a method according to any one of claims 1 to 14 to obtain indications of the relative abundances of bacteria within the first sample and the second sample;
comparing the indications of the relative abundances of the bacteria within the first sample with the relative abundance of the bacteria in the second sample;
selecting one or more bacteria with a genome, which is present in the first sample but absent in the second sample for bacteriotherapy for the disease.

18. A method of diagnosing a disease in a patient, the method comprising:
obtaining a plurality of sequence reads from a patient sample obtained from the patient;
using a method according to any one of claims 1 to 14 to obtain indications of the relative abundances of microorganisms within the patient sample;
comparing the indications of the relative abundances of the microorganisms within the patient sample with the relative abundance of the microorganisms in a control;
identifying one or more microorganisms with a genome, which is present in the patient sample but absent from the control, or which is present at a higher abundance in the patient sample compared with the control or which is present at a lower level in the patient sample compared with the control;
wherein the presence, or higher abundance, or lower level of the one or more microorganisms in the patient sample is indicative of the disease.

## Patentansprüche

1. Verfahren zum Analysieren von Mikroorganismen, die in einer Probe vorhanden sind, unter Verwendung einer Datenbank, die eine Vielzahl mikrobieller Referenzgenome für die Mikroorganismen und phylogenetische Informationen speichert, die die gespeicherten Referenzgenome in einer phylogenetischen Struktur miteinander in Beziehung setzt, wobei das Verfahren einschließt:
Verwenden einer Vielzahl von Sequenzablesungen, die aus einer Probe erhalten wurden, um die Anzahl der Sequenzablesungen zu zählen, die als eindeutig auf jedes von einer Vielzahl von Referenzgenomen innerhalb der phylogenetischen Struktur gemappt angesehen werden, mittels folgendem:
für das/jedes Referenzgenom in der Vielzahl der Referenzgenome:
Vergleichen der Vielzahl von Sequenzablesungen mit dem Referenzgenom, um herzuleiten, ob jedwede Sequenzablesungen auf das Referenzgenom und kein sonstiges Referenzgenom, das in der Datenbank gespeichert ist, gemappt sind;
falls eine Sequenzablesung auf das Referenzgenom und kein sonstiges Referenzgenom, das in der Datenbank gespeichert ist, gemappt ist, Zählen der Sequenzablesung als eindeutig auf das Referenzgenom gemappt angesehen; und
für jedes von der Vielzahl der Referenzgenome, auf die mindestens eine Sequenzablesung als eindeutig gemappt angesehen worden ist, Normalisieren der Anzahl der Sequenzablesungen, die als eindeutig auf das Referenzgenom gemappt angesehen gezählt wurden, indem die gezählte Anzahl der Sequenzablesungen durch ein Maß geteilt wird, das die Eindeutigkeit des Referenzgenoms widerspiegelt, und Vergleichen der normalisierten Anzahl von Sequenzablesungen mit der normalisierten Anzahl von Sequenzablesungen für die sonstigen Referenzgenome, um so eine Angabe der relativen Häufigkeit der mikrobiellen Referenzgenome der Mikroorganismen innerhalb der Probe zu erhalten,
wobei das Maß, das die Eindeutigkeit des Referenzgenoms widerspiegelt, wie folgt bestimmt wird:
für das/jedes Referenzgenom:
Identifizieren von einer oder mehreren genetischen Sequenzen, die als das Referenzgenom eindeutig identifizierend angesehen werden; und
Bestimmen des Maßes, das die Eindeutigkeit des Referenzgenoms widerspiegelt, indem die kombinierte Länge der einen oder mehreren genetischen Sequenzen, die als das Referenzgenom eindeutig identifizierend angesehen werden, durch die Länge des entsprechenden Referenzgenoms geteilt wird, und
wobei Identifizieren der einen oder mehreren genetischen Sequenzen, die als jedes von der Vielzahl der Referenzgenome eindeutig identifizierend angesehen werden, einschließt:
für jedes Referenzgenom in der Datenbank:
Definieren einer Vielzahl von Segmenten, wobei jedes Segment eine von dem Referenzgenom verschiedene genetische Sequenz enthält;
für die in jedem Segment enthaltene genetische Sequenz:
Vergleichen der in dem Segment enthaltenen genetischen Sequenz mit der Gesamtheit des genetischen Gehalts aller sonstigen Referenzgenome in der Datenbank, um herzuleiten, ob das Segment auf jedwedes der sonstigen Referenzgenome gemappt ist, wobei Herleiten, ob das Segment auf jedwedes der sonstigen Referenzgenome gemappt ist, geringfügige Differenzen von zwei bis drei Basenpaaren für ein Segment mit 100 Basenpaaren Länge ignoriert; und
falls die in dem Segment enthaltene genetische Sequenz auf kein sonstiges Referenzgenom in der Datenbank gemappt ist, Identifizieren der in dem Segment enthaltenen genetischen Sequenz als eindeutig das Referenzgenom identifizierend angesehen.

2. Verfahren nach Anspruch 1, wobei die Vielzahl von Segmenten, die für jedes Referenzgenom definiert ist, eine vorbestimmte Länge hat und jedes mögliche Segment mit jener Länge eingeschlossen ist, das für das Referenzgenom definiert sein könnte.

3. Verfahren nach einem der vorhergehenden Ansprüche, wobei die Datenbank einen Eintrag für jedes Referenzgenom innerhalb der phylogenetischen Struktur einschließt, und vorzugsweise der Eintrag für jedes Referenzgenom ein Elternfeld zum Speichern eines Zeigers auf ein Elternteil des Referenzgenoms innerhalb der phylogenetischen Struktur einschließt.

4. Verfahren nach Anspruch 3, wobei der Eintrag für jedes Referenzgenom ein Eindeutigkeitsfeld zum Speichern des Maßes einschließt, welches die Eindeutigkeit des Referenzgenoms widerspiegelt.

5. Verfahren nach Anspruch 4, wobei das Eindeutigkeitsfeld jedes Mal, wenn ein neues Referenzgenom in der Datenbank gespeichert wird, erneut berechnet wird.

6. Verfahren nach einem der vorhergehenden Ansprüche, wobei die Sequenzablesungen mittels eines Shotgun-Sequenzierungsprozesses erhalten werden, bei dem die in der Probe enthaltene DNA statistisch in kleine Segmente aufgebrochen wird, die dann sequenziert werden, um die Vielzahl der Sequenzablesungen zu erhalten, wobei die Vielzahl der Sequenzablesungen aus der Probe über die gesamte DNA der Organismen innerhalb der Probe erhalten wird.

7. Verfahren nach einem der vorhergehenden Ansprüche, wobei die Sequenzablesungen jeweils eine Länge von mindestens 80 oder mehr Basenpaaren haben.

8. Verfahren nach einem der vorhergehenden Ansprüche, einschließend:
Erhalten einer Vielzahl von Sequenzablesungen aus einem ersten Anteil der Probe, und Erhalten von Angaben der relativen Häufigkeiten von einem oder mehreren Mikroorganismen innerhalb des ersten Anteils der Probe;
Erhalten einer Vielzahl von Sequenzablesungen aus Mikroorganismen, die aus einem zweiten Anteil der Probe unter Verwendung eines mikrobiellen Kulturverfahrens kultiviert wurden, und Erhalten von Angaben der relativen Häufigkeiten von einem oder mehreren Mikroorganismen innerhalb des kultivierten Anteils der Probe; und
Vergleichen der Angaben der relativen Häufigkeiten der Mikroorganismen innerhalb des ersten Anteils der Probe mit den Angaben der relativen Häufigkeit der Mikroorganismen innerhalb des zweiten Anteils der Probe.

9. Verfahren nach Anspruch 8, ferner umfassend:
Erhalten einer Vielzahl von Sequenzablesungen aus Mikroorganismen, die aus einem dritten Anteil der Probe, der aus derselben Quelle wie der erste und der zweite Anteil der Proben erhalten wurde, unter Verwendung eines alternativen mikrobiellen Kulturverfahrens kultiviert wurden, und Erhalten von Angaben der relativen Häufigkeiten des einen oder der mehreren Mikroorganismen innerhalb des dritten Anteils der Probe; und
Vergleichen der Angaben der relativen Häufigkeiten der Mikroorganismen innerhalb des dritten Anteils der Probe mit den Angaben der relativen Häufigkeit der Mikroorganismen innerhalb mindestens eines von dem ersten und dem zweiten Anteil der Proben.

10. Verfahren nach Anspruch 8, einschließend:
Bestimmen eines interessierenden Mikroorganismus, der in der Probe vorhanden ist, die unter Verwendung des mikrobiellen Kulturverfahrens kultiviert wurden, indem die Angaben der relativen Häufigkeiten der Mikroorganismen innerhalb des ersten Anteils der Probe mit den Angaben der relativen Häufigkeit der Mikroorganismen innerhalb des zweiten Anteils der Probe verglichen werden;
Verwenden des mikrobiellen Kulturverfahrens, um Kulturen des einen oder der mehreren der interessierenden Mikroorganismen aus der Probe zu präparieren, und
Bestimmen der genomischen Sequenz(en) dieser Mikroorganismen.

11. Verfahren nach Anspruch 9, ferner umfassend Zufügen der genomischen Sequenz(en) des einen oder der mehreren der interessierenden Mikroorganismen zu der Datenbank, die Referenzgenome speichert.

12. Verfahren nach einem der Ansprüche 1 bis 11, wobei die Probe von einem Menschen erhalten wird.

13. Verfahren nach einem der Ansprüche 1 bis 12, des Weiteren umfassend Verwenden der Datenbank zum Korrelieren der erhaltenen relativen Häufigkeit der Mikroorganismen innerhalb der Probe mit Gesundheits- oder Erkrankungsstatus des Wirts, von dem die Probe erhalten wurde, indem die erhaltene relative Häufigkeit der Mikroorganismen in Proben mit einer speziellen Erkrankung mit gesunden Proben in der Datenbank verglichen wird.

14. Verfahren nach einem der Ansprüche 1 bis 13, wobei der Mikroorganismus ein Bakterium ist.

15. Verfahren zum Identifizieren von einem oder mehreren Bakterien für die Bakteriotherapie einer Dysbiose oder einer Erkrankung, wobei das Verfahren umfasst:
Erhalten einer Vielzahl von Sequenzablesungen aus einer Probe, die von einem Patienten mit der Dysbiose oder Erkrankung erhalten wurde;
Verwenden eines Verfahrens gemäß einem der Ansprüche 1 bis 14, um Angaben der relativen Häufigkeiten von Bakterien innerhalb der Probe zu erhalten;
Vergleichen der Angaben der relativen Häufigkeiten der Bakterien innerhalb der Probe mit der relativen Häufigkeit der Bakterien in einer Kontrolle;
Auswählen von einem oder mehreren Bakterien mit einem Genom, das in der von dem Patienten erhaltene Probe fehlt, jedoch in der Kontrolle vorhanden ist, oder das in der von dem Patienten erhaltenen Probe in einer geringeren relativen Häufigkeit vorhanden ist, verglichen mit der Kontrolle, zur Bakteriotherapie der Dysbiose oder der Erkrankung.

16. Verfahren zum Identifizieren von einem oder mehreren Bakterien für die Bakteriotherapie einer Dysbiose oder einer Erkrankung, wobei das Verfahren umfasst:
Erhalten einer Vielzahl von Sequenzablesungen aus (i) einer ersten Probe, die von einem Patienten mit der Dysbiose oder Erkrankung erhalten wurde;
Erhalten einer Vielzahl von Sequenzablesungen aus (ii) einer zweiten Probe, die von demselben Patienten erhalten wurde, nachdem der Patient eine Fäkaltransplantation erhalten hat;
Verwenden eines Verfahrens gemäß einem der Ansprüche 1 bis 14, um Angaben der relativen Häufigkeiten von Bakterien innerhalb der ersten und zweiten Probe zu erhalten;
Vergleichen der Angaben der relativen Häufigkeiten der Bakterien innerhalb der ersten Probe mit der relativen Häufigkeit der Bakterien in der zweiten Probe;
Auswählen von einem oder mehreren Bakterien mit einem Genom, das in der ersten Probe fehlt, jedoch in der zweiten Probe vorhanden ist, oder das in der ersten Probe, verglichen mit der zweiten Probe, in einer geringeren relativen Häufigkeit vorhanden ist, zur Bakteriotherapie der Dysbiose oder der Erkrankung.

17. Verfahren zum Identifizieren von einem oder mehreren Bakterien zur Therapie einer Erkrankung, die durch die Anwesenheit eines pathogenen Bakteriums charakterisiert ist, wobei ein asymptomatischer Träger des pathogenen Bakteriums jedoch keine Erkrankungssymptome dafür zeigt, wobei das Verfahren umfasst:
Erhalten einer Vielzahl von Sequenzablesungen aus einer ersten Probe, die von einem asymptomatischen Träger des pathogenen Bakteriums erhalten wurde, und Erhalten einer Vielzahl von Sequenzablesungen aus einer zweiten Probe, die von einem gesunden Individuum erhalten wurde;
Verwenden eines Verfahrens gemäß einem der Ansprüche 1 bis 14, um Angaben der relativen Häufigkeiten von Bakterien innerhalb der ersten Probe und der zweiten Probe zu erhalten;
Vergleichen der Angaben der relativen Häufigkeiten der Bakterien innerhalb der ersten Probe mit der relativen Häufigkeit der Bakterien in der zweiten Probe;
Auswählen von einem oder mehreren Bakterien mit einem Genom, das in der ersten Probe vorhanden ist, jedoch in der zweiten Probe fehlt, zur Bakteriotherapie der Erkrankung.

18. Verfahren zum Diagnostizieren einer Erkrankung bei einem Patienten, wobei das Verfahren umfasst:
Erhalten einer Vielzahl von Sequenzablesungen aus einer Patientenprobe, die von dem Patienten erhalten wurde;
Verwenden eines Verfahrens gemäß einem der Ansprüche 1 bis 14, um Angaben der relativen Häufigkeiten von Mikroorganismen innerhalb der Patientenprobe zu erhalten;
Vergleichen der Angaben der relativen Häufigkeiten der Mikroorganismen innerhalb der Patientenprobe mit der relativen Häufigkeit der Mikroorganismen in einer Kontrolle;
Identifizieren von einem oder mehreren Mikroorganismen mit einem Genom, das in der Patientenprobe vorhanden ist, jedoch in der Kontrolle fehlt, oder das in der Patientenprobe, verglichen mit der Kontrolle, in einer höheren Häufigkeit vorhanden ist, oder das in der Patientenprobe, verglichen mit der Kontrolle, in einem niedrigeren Niveau vorhanden ist;
wobei die Anwesenheit oder höhere Häufigkeit oder das niedrigere Niveau des einen oder der mehreren Mikroorganismen in der Patientenprobe Aufschluss über die Erkrankung gibt.

## Revendications

1. Procédé d'analyse de micro-organismes présents dans un échantillon au moyen d'une base de données qui stocke une pluralité de génomes microbiens de référence pour les micro-organismes et des informations phylogénétiques qui lient les génomes de référence stockés les uns aux autres dans une structure phylogénétique, le procédé comportant :
l'utilisation d'une pluralité de lectures de séquence obtenues à partir d'un échantillon pour compter le nombre de lectures de séquence jugées correspondre de façon unique à chacun d'une pluralité de génomes de référence à l'intérieur de la structure phylogénétique par :
pour le/chaque génome de référence dans la pluralité de génomes de référence :
comparaison de la pluralité de lectures de séquence avec le génome de référence pour établir si des lectures de séquence quelles qu'elles soient correspondent au génome de référence et ne correspondent à aucun autre génome de référence stocké dans la base de données ;
si une lecture de séquence correspond au génome de référence et ne correspond à aucun autre génome de référence stocké dans la base de données, comptage de la lecture de séquence comme jugée correspondre de façon unique au génome de référence ; et
pour chacun de la pluralité de génomes de référence auxquels au moins une lecture de séquence a été jugée correspondre de façon unique, la normalisation du nombre de lectures de séquence comptées comme jugées correspondre de façon unique au génome de référence par division du nombre compté de lectures de séquence par une mesure qui reflète le caractère unique du génome de référence et comparaison du nombre normalisé de lectures de séquence au nombre normalisé de lectures de séquence pour les autres génomes de référence de manière à obtenir une indication de l'abondance relative des génomes microbiens de référence des micro-organismes à l'intérieur de l'échantillon,
dans lequel la mesure qui reflète le caractère unique du génome de référence est déterminée par :
pour le/chaque génome de référence :
identification d'une ou plusieurs séquences génétiques jugées identifier de façon unique le génome de référence ; et
détermination de la mesure qui reflète le caractère unique du génome de référence par division de la longueur combinée de la ou des séquences génétiques jugées identifier de façon unique le génome de référence par la longueur du génome de référence correspondant, et
dans lequel l'identification d'une ou plusieurs séquences génétiques jugées identifier de façon unique chacun de la pluralité de génomes de référence comporte,
pour chaque génome de référence dans la base de données :
la définition d'une pluralité de segments, chaque segment contenant une séquence génétique différente issue du génome de référence ;
pour la séquence génétique contenue dans chaque segment :
la comparaison de la séquence génétique contenue dans le segment avec la totalité du contenu génétique de tous les autres génomes de référence dans la base de données pour établir si le segment correspond à l'un quelconque des autres génomes de référence, l'établissement que le segment correspond ou non à l'un quelconque des autres génomes de référence ignorant les différences mineures de deux ou trois paires de bases pour un segment dont la longueur s'élève à 100 paires de bases ; et
si la séquence génétique contenue dans le segment ne correspond à aucun autre génome de référence dans la base de données, l'identification de la séquence génétique contenue dans le segment comme jugée identifier de façon unique le génome de référence.

2. Procédé selon la revendication 1, dans lequel la pluralité de segments définis pour chaque génome de référence ont une longueur prédéterminée, et incluent chaque segment possible de cette longueur qui pourrait être défini pour le génome de référence.

3. Procédé selon une quelconque revendication précédente, dans lequel la base de données comporte une entrée pour chaque génome de référence à l'intérieur de la structure phylogénétique, et de préférence l'entrée pour chaque génome de référence comporte un champ parent destiné à stocker un pointeur vers un parent du génome de référence à l'intérieur de la structure phylogénétique.

4. Procédé selon la revendication 3, dans lequel l'entrée pour chaque génome de référence comporte un champ caractère unique destiné à stocker la mesure qui reflète le caractère unique du génome de référence.

5. Procédé selon la revendication 4, dans lequel le champ caractère unique est recalculé chaque fois qu'un nouveau génome de référence est stocké dans la base de données.

6. Procédé selon une quelconque revendication précédente, dans lequel les lectures de séquence sont obtenues par un processus de séquençage en aveugle dans lequel l'ADN contenu dans l'échantillon est cassé aléatoirement en petits segments qui sont ensuite séquencés pour obtenir la pluralité de lectures de séquence, dans lequel la pluralité de lectures de séquence issues de l'échantillon sont obtenues d'un bout à l'autre de l'ADN complet d'organismes à l'intérieur de l'échantillon.

7. Procédé selon une quelconque revendication précédente, dans lequel les lectures de séquence ont chacune une longueur d'au moins 80 paires de bases ou plus.

8. Procédé d'une quelconque revendication précédente comportant :
l'obtention d'une pluralité de lectures de séquence à partir d'une première portion de l'échantillon et l'obtention d'indications des abondances relatives d'un ou plusieurs micro-organismes à l'intérieur de la première portion de l'échantillon ;
l'obtention d'une pluralité de lectures de séquence issues de micro-organismes cultivés à partir d'une deuxième portion de l'échantillon au moyen d'un procédé de culture microbienne et l'obtention d'indications des abondances relatives d'un ou plusieurs micro-organismes à l'intérieur de la portion cultivée de l'échantillon ; et
la comparaison des indications des abondances relatives des micro-organismes à l'intérieur de la première portion de l'échantillon avec les indications de l'abondance relative des micro-organismes à l'intérieur de la deuxième portion de l'échantillon.

9. Procédé selon la revendication 8, comprenant en outre :
l'obtention d'une pluralité de lectures de séquence issues de micro-organismes cultivés à partir d'une troisième portion de l'échantillon, obtenue à partir de la même source que les première et deuxième portions des échantillons au moyen d'un procédé de culture microbienne alternatif, et l'obtention d'indications des abondances relatives d'un ou plusieurs micro-organismes à l'intérieur de la troisième portion de l'échantillon ; et
la comparaison des indications des abondances relatives des micro-organismes à l'intérieur de la troisième portion de l'échantillon avec les indications de l'abondance relative des micro-organismes à l'intérieur d'au moins une des première et deuxième portions des échantillons.

10. Procédé selon la revendication 8, comportant :
la détermination d'un micro-organisme d'intérêt présent dans l'échantillon qui a été cultivé au moyen du procédé de culture microbienne par comparaison des indications des abondances relatives des micro-organismes à l'intérieur de la première portion d'échantillon avec les indications de l'abondance relative des micro-organismes à l'intérieur de la deuxième portion de l'échantillon ;
l'utilisation du procédé de culture microbienne pour préparer des cultures d'un ou plusieurs des micro-organismes d'intérêt à partir de l'échantillon ; et
la détermination de la/des séquence(s) génomique(s) dudit micro-organisme.

11. Procédé selon la revendication 9, comprenant en outre l'ajout de la/des séquence(s) génomique(s) dudit ou desdits micro-organismes d'intérêt à la base de données qui stocke des génomes de référence.

12. Procédé selon l'une quelconque des revendications 1 à 11, dans lequel l'échantillon est obtenu auprès d'un humain.

13. Procédé selon l'une quelconque des revendications 1 à 12, comprenant en outre l'utilisation de la base de données pour corréler l'abondance relative obtenue des micro-organismes à l'intérieur de l'échantillon avec la santé ou l'état maladif de l'hôte auprès duquel l'échantillon a été obtenu en comparant l'abondance relative obtenue des micro-organismes dans des échantillons avec une maladie particulière avec des échantillons sains dans la base de données.

14. Procédé selon l'une quelconque des revendications 1 à 13, dans lequel le micro-organisme est une bactérie.

15. Procédé d'identification d'une ou plusieurs bactéries pour de la bactériothérapie pour une dysbiose ou une maladie, le procédé comprenant :
l'obtention d'une pluralité de lectures de séquence à partir d'un échantillon obtenu auprès d'un patient avec la dysbiose ou la maladie ;
l'utilisation d'un procédé selon l'une quelconque des revendications 1 à 14 pour obtenir des indications des abondances relatives de bactéries à l'intérieur de l'échantillon ;
la comparaison des indications des abondances relatives des bactéries à l'intérieur de l'échantillon avec l'abondance relative des bactéries dans un témoin ;
la sélection d'une ou plusieurs bactéries avec un génome qui est absent de l'échantillon obtenu auprès du patient, mais présent dans le témoin, ou qui est présent avec une abondance relative moindre dans l'échantillon obtenu auprès du patient comparé au témoin, pour de la bactériothérapie pour la dysbiose ou la maladie.

16. Procédé d'identification d'une ou plusieurs bactéries pour de la bactériothérapie pour une dysbiose ou une maladie, le procédé comprenant :
l'obtention d'une pluralité de lectures de séquence à partir (i) d'un premier échantillon obtenu auprès d'un patient avec la dysbiose ou la maladie ;
l'obtention d'une pluralité de lectures de séquence à partir (ii) d'un deuxième échantillon obtenu auprès du même patient après que le patient a reçu un transplant fécal ;
l'utilisation d'un procédé selon l'une quelconque des revendications 1 à 14 pour obtenir des indications des abondances relatives de bactéries à l'intérieur des premier et deuxième échantillons ;
la comparaison des indications des abondances relatives des bactéries à l'intérieur du premier échantillon avec l'abondance relative des bactéries dans le deuxième échantillon ;
la sélection d'une ou plusieurs bactéries avec un génome qui est absent du premier échantillon, mais présent dans le deuxième échantillon, ou qui est présent avec une abondance relative moindre dans le premier échantillon comparé au deuxième échantillon, pour de la bactériothérapie pour la dysbiose ou la maladie.

17. Procédé d'identification d'une ou plusieurs bactéries pour le traitement d'une maladie qui se **caractérise par** la présence d'une bactérie pathogène, mais pour laquelle aucun symptôme maladif n'est présenté par un porteur asymptomatique de la bactérie pathogène, le procédé comprenant :
l'obtention d'une pluralité de lectures de séquence à partir d'un premier échantillon obtenu auprès d'un porteur asymptomatique de la bactérie pathogène et l'obtention d'une pluralité de lectures de séquence à partir d'un deuxième échantillon obtenu auprès d'un individu sain ;
l'utilisation d'un procédé selon l'une quelconque des revendications 1 à 14 pour obtenir des indications des abondances relatives de bactéries à l'intérieur du premier échantillon et du deuxième échantillon ;
la comparaison des indications des abondances relatives des bactéries à l'intérieur du premier échantillon avec l'abondance relative des bactéries dans le deuxième échantillon ;
la sélection d'une ou plusieurs bactéries avec un génome qui est présent dans le premier échantillon, mais absent du deuxième échantillon, pour de la bactériothérapie pour la maladie.

18. Procédé de diagnostic d'une maladie chez un patient, le procédé comprenant :
l'obtention d'une pluralité de lectures de séquence à partir d'un échantillon patient obtenu auprès du patient ;
l'utilisation d'un procédé selon l'une quelconque des revendications 1 à 14 pour obtenir des indications des abondances relatives de micro-organismes à l'intérieur de l'échantillon patient ;
la comparaison des indications des abondances relatives des micro-organismes à l'intérieur de l'échantillon patient avec l'abondance relative des micro-organismes dans un témoin ;
l'identification d'un ou plusieurs micro-organismes avec un génome qui est présent dans l'échantillon patient, mais absent du témoin, ou qui est présent avec une plus grande abondance dans l'échantillon patient comparé au témoin, ou qui est présent à un niveau moindre dans l'échantillon patient comparé au témoin ;
dans lequel la présence, ou la plus grande abondance, ou le niveau moindre du ou des micro-organismes dans l'échantillon patient constitue une indication de la maladie.
